# FASCICULE DE BREVET EUROPEEN

(11) **EP 0 745 583 B1**
(45) Date de publication et mention de la délivrance du brevet: **29.12.1999**
(21) Numéro de dépôt: 96401158.9
(22) Date de dépôt: 30.05.1996
(51) Int. Cl.: C07C 233/18, C07C 275/24, C07C 335/12, C07D 209/16, C07D 333/58, C07D 307/81, A61K 31/165, A61K 31/17, A61K 31/40, A61K 31/38, A61K 31/34

(54) **Composés alkoxy-aryles ayant une affinité pour les récepteurs mélatoninergiques, leur procédé de préparation, et les compositions pharmaceutiques qui les contiennent**
Alkoxyarylverbindungen mit Affinität zu den Melatoninrezeptoren, Verfahren zu ihrer Herstellung und sie enthaltende pharmazeutische Zusammensetzungen
Alkoxy aryl compounds with affinity to the melatonine receptors, process for their preparation and pharmaceutical compositions containing them

(30) Priorité: 31.05.1995 FR 9506434
(43) Date de publication de la demande: 04.12.1996
(73) Titulaire: ADIR ET COMPAGNIE, 92415 Courbevoie Cédex (FR)
(72) Inventeur: Lesieur, Daniel, 59147 Gondecourt (FR); Depreux, Patrick, 59280 Armentieres (FR); Leclerc, Véronique, 51000 Lille (FR); Delagrance, Philippe, 92130 Issy-les-Moulineaux (FR); Renard, Pierre, 78000 Versailles (FR)

(56) Documents cités:
- EP-A- 0 447 285
- EP-A- 0 527 687
- EP-A- 0 530 087
- JOURNAL OF MEDICINAL CHEMISTRY, vol. 22, no. 1, WASHINGTON US, pages 63-69, XP002011140 M. E. FLAUGH ET AL.: "Synthesis and evaluation of the antiovulatory activity of a variety of melatonin analogues"

## Description

L'invention conceme de nouveaux composés alkoxy-aryles, leur procédé de préparation et les compositions pharmaceutiques qui les contiennent.

De nombreuses études ont mis en évidence ces dix demières années, le rôle capital de la mélatonine (5-méthoxy N-acétyl tryptamine) dans le contrôle du rythme circadien et des fonctions endocrines, et les récepteurs de la mélatonine ont été caractérisés et localisés.

Outre leur action bénéfique sur les troubles du rythme circadien (J. Neurosurg 1985, 63, pp 321-341) et du sommeil (Psychopharmacology, 1990, 100, pp 222-226), les ligands du système mélatoninergique possèdent d'intéressantes propriétés pharmacologiques sur le système nerveux central, notamment anxiolytiques et antipsychotiques (Neuropharmacology of Pineal Secretions, 1990, 8 (3-4), pp 264-272) et analgésiques (Pharmacopsychiat., 1987, 20, pp 222-223) ainsi que pour le traitement des maladies de Parkinson (J. Neurosurg 1985, 63, pp 321-341) et d'Alzheimer (Brain Research, 1990, 528, pp 170-174). De même, ces composés ont montré une activité sur certains cancers (Melatonin - clinical Perspectives, Oxford University Press, 1988, page 164-165), sur l'ovulation (Science 1987, 227, pp 714-720) et sur le diabète (Clinical endocrinolgy, 1986, 24, pp 359-364).

Des composés permettant d'agir sur le système mélatoninergique sont donc pour le clinicien d'excellents médicaments pour le traitement des pathologies liées au système mélatoninergique et notamment celles mentionnées précédemment.

La demanderesse a découvert de nouveaux composés alkoxy-aryles, de structure originale, montrant une très haute affinité pour les récepteurs mélatoninergiques et présentant, in vitro et in vivo, un grand intérêt pharmacologique et thérapeutique.

L'invention concerne plus particulièrement les composés de formule (I) : 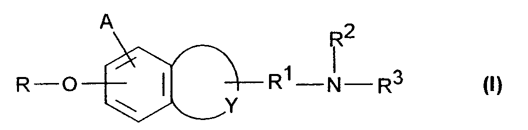 dans laquelle :
- R¹ représente une chaîne (C₁-C₄) alkylène non substituée ou substituée par un radical choisi parmi alkyle, hydroxy, alkoxycarbonyle et carboxyle ;
- R² représente un atome d'hydrogène ou un alkyle ;
- R³ représente :
   . soit un groupement de formule R³¹ 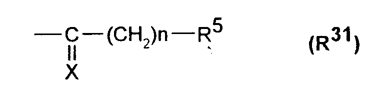 dans lequel n représente zéro ou un nombre entier de 1 à 3, X représente un soufre ou un oxygène et R⁵ représente un atome d'hydrogène, un alkyle non substitué ou substitué, un alcényle, un alcynyle, un cycloalkyle non substitué ou substitué, un dicycloalkylalkyle non substitué ou substitué,
   . soit un groupement de formule R³² : 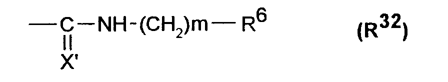 dans lequel X' représente un atome d'oxygène ou de soufre,
      m représente zéro ou un nombre entier de 1 à 3
      et R⁶ représente un atome d'hydrogène, un alkyle non substitué ou substitué, un alcényle ou un alcynyle, étant entendu que R⁶ peut également représenter un cycloalkyle non substitué ou substitué ou un dicycloalkylalkyl non substitué ou substitué lorsque X' est un oxygène;
- A représente un radical choisi parmi un alkyle non substitué ou substitué et un groupement A' choisi parmi un alcényle ou un alcynyle,
- R représente un groupement choisi parmi un alkyle non substitué ou substitué, un alcényle, un alcynyle, un cycloalkyle non substitué ou substitué, un cycloalkylalkyle non substitué ou substitué, un aryle non substitué ou substitué, un arylalkyle non substitué ou substitué, un diarylalkyle non substitué ou substitué, un cycloalcényle et un cycloalcénylalkyle,
Y forme avec le noyau benzo auquel il est lié un groupement Y¹ qui
- soit représente un groupement choisi parmi naphtalène, benzofurane, benzothiophène et indole, dans le cas où A représente un groupement A',
- soit représente un groupement naphtalène, dans le cas où A représente un radical alkyle non substitué ou substitué,
Y¹ étant éventuellement partiellement hydrogéné,
étant entendu que :
- l'expression "substitué" affectant le terme "alkyle" signifie que ce groupement est substitué par un ou plusieurs radicaux choisis parmi halogène, hydroxy, et alkoxy,
- l'expression "substitué" affectant les termes "cycloalkyle", "cycloalkylalkyle" et "dicycloalkylalkyle" signifie que ces groupements sont substitués sur la partie cycloalkyle par un ou plusieurs groupements choisis parmi halogène, alkyle, alkoxy, hydroxy et oxo,
- l'expression "substitué" affectant les termes "aryle", "arylalkyle" et "diarylalkyle" signifie que ces groupements sont substitués sur le noyau aromatique par un ou plusieurs radicaux choisis parmi halogène, alkyle, alkyle substitué par un ou plusieurs halogènes, alkoxy et hydroxy,
- les termes "alkyle" et "alkoxy" désignent des radicaux linéaires ou ramifiés comportant de 1 à 6 atomes de carbone,
- les termes "alcényle" et "alcynyle" désignent des radicaux linéaires ou ramifiés insaturés de 2 à 6 atomes de carbone,
- le terme "cycloalkyle" désigne un groupement cyclique de 3 à 8 atomes de carbone, saturé,
- le terme "cycloalcényle" désigne un groupement cyclique insaturé de 3 à 8 atomes de carbone,
- le terme "aryle" désigne un groupement phényle ou naphtyle,
leurs énantiomères et diastéréoisomères,
et leurs sels d'addition à une base pharmaceutiquement acceptable.
Particulièrement, l'invention concerne :
- les composés de formule (I) dans laquelle R¹ représente une chaîne éthylène,
- les composés de formule (I) dans laquelle R² représente un atome d'hydrogène,
- les composés de formule (I) dans laquelle R³ représente un groupement de formule R³¹,
- les composés de formule (I) dans laquelle R⁵ représente un alkyle,
- les composés de formule (I) dans laquelle R⁵ représente un groupement cycloalkyle,
- les composés de formule (I) dans laquelle R³ représente un groupement R³²,
- les composés de formule (I) dans laquelle R⁶ représente un alkyle,
- les composés de formule (I) dans laquelle R⁶ représente un cycloalkyle,
- les composés de formule (I) dans laquelle X est un atome d'oxygène,
- les composés de formule (I) dans laquelle X est un atome de soufre,
- les composés de formule (I) dans laquelle X' est un atome d'oxygène,
- les composés de formule (I) dans laquelle X' est un atome de soufre,
- les composés de formule (I) dans laquelle A est un radical alkyle,
- les composés de formule (I) dans laquelle A est un radical A',
- les composés de formule (I) dans laquelle A' est un radical alcényle,
- les composés de formule (I) dans laquelle A' est un radical alcynyle,
- les composés de formule (I) dans laquelle R est un radical alkyle,
- les composés de formule (I) dans laquelle R est un radical alcényle,
- les composés de formule (I) dans laquelle R est un radical alcynyle,
- les composés de formule (I) dans laquelle Y forme avec le noyau benzo auquel il est lié, un groupement naphtalène,
- les composés de formule (I) dans laquelle Y forme avec le noyau benzo auquel il est lié, un groupement tétrahydronaphtalène,
- les composés de formule (I) dans laquelle Y forme avec le noyau benzo auquel il est lié, un groupement indole,
- et les composés de formule (I) dans laquelle Y forme avec le noyau benzo auquel il est lié, un groupement benzofurane ou benzothiophène.
Plus particulièrement, l'invention concerne :
- les composés de formule (I₁) 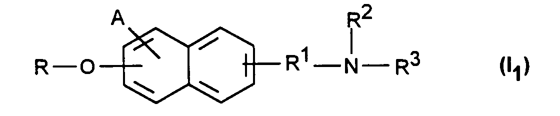 dans laquelle A, R, R¹, R² et R³ sont tels que définis dans la formule (I),
- les composés de formule (I₂) 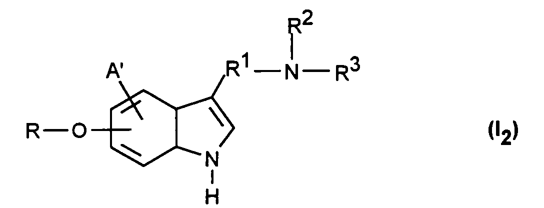 dans laquelle A', R, R¹, R² et R³ sont tels que définis dans la formule (I),
   et les composés de formule (I₃) : 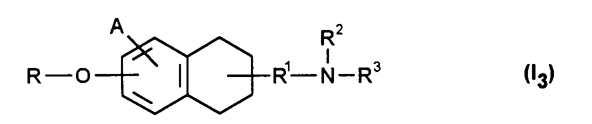 dans laquelle A, R, R¹, R² et R³ sont tels que définis dans la formule (I).

Par exemple, l'invention concerne les composés de formule (I₄) : 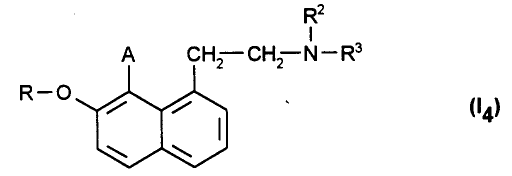 dans laquelle A, R, R² et R³ sont tels que définis dans la formule (I), les composés de formule (I₅) : 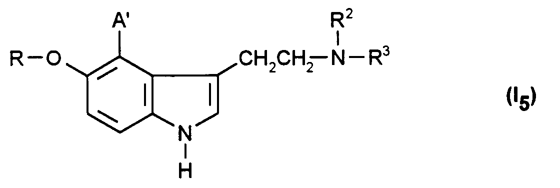 dans laquelle A', R, R² et R³ sont tels que définis dans la formule (I), et les composés de formule (I₆) : 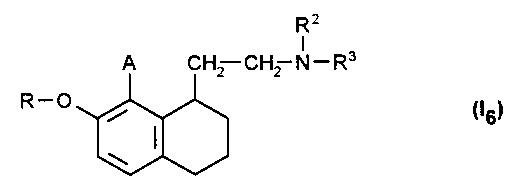 dans laquelle A, R, R² et R³ sont tels que définis dans la formule (I).

De façon particulière, les radicaux alkyles présents dans la formule (I) peuvent être choisis parmi méthyle, éthyle, n-propyle, isopropyle, n-butyle, isobutyle, sec-butyle, tert-butyle, pentyle et, hexyle et les isomères de squelette des radicaux pentyle et hexyle,
les radicaux alcényles présents dans la formule (I) peuvent être chosis parmi vinyle, allyle, propényle, butényle, pentényle et hexényle et leurs isomères selon la position de la double liaison,
les radicaux alcynyles présents dans la formule (I) peuvent être choisis parmi éthynyle, propargyle, butynyle, pentynyle et hexynyle, et leurs isomères selon la position de la triple liaison,
les radicaux alkoxy présents dans la formule (I) peuvent être choisis parmi méthoxy, éthoxy, n-propoxy, isopropoxy, n-butoxy, iso-butoxy, sec-butoxy, tert-butoxy, pentyloxy et hexyloxy et les isomères de squelette des radicaux pentyloxy et hexyloxy,
les halogènes présents dans la formule (I) peuvent être choisis parmi le brome, le chlore, le fluor et l'iode,
les cycloalkyles présents dans la formule (I) peuvent être choisis parmi cyclopropyle, cyclobutyle, cyclopentyle, cyclohexyle, cycloheptyle et cyclooctyle,
le cycloalcényles présents dans la formule (I) peuvent être choisis parmi cyclopropényle, cyclobutényle, cyclopentényle, cyclohexényle, cycloheptényle et cyclooctényle,
et les groupements alkylène présents dans la formule (I) peuvent être choisis parmi méthylène, éthylène, triméthylène, tétraméthylène, pentaméthylène et hexaméthylène.

Parmi les bases pharmaceutiquement acceptables que l'on peut utiliser pour former un sel d'addition avec les composés de l'invention, on peut citer à titre d'exemples et de façon non limitative, les hydroxydes de sodium, de potassium, de calcium, ou d'aluminium, les carbonates de métaux alcalins ou alcalinoterreux, et les bases organiques comme la triéthylamine, la benzylamine, la diéthanolamine, la tert-butylamine, la dicyclohexylamine, et l'arginine.

L'invention conceme le procédé de préparation des composés de formule (I) caractérisé en ce qu'on fait réagir un composé de formule (II): 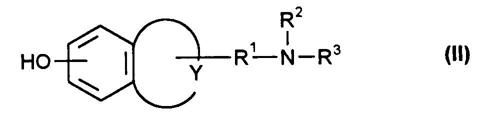 dans laquelle R¹, R² et R³ et Y sont tels que définis dans la formule (I),
avec un composé de formule (III/a) :

A―Z (III/a)

où A a la même définition que dans la formule (I) et Z représente un groupement partant, par exemple un atome d'halogène ou un groupement tosyle,
afin d'obtenir un composé de formule (IV) : 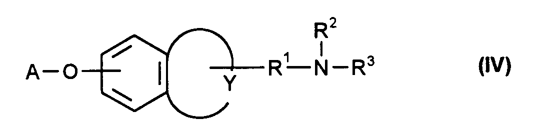 dans laquelle A, R¹, R², R³ et Y sont tels que définis précédemment, ce composé de formule (IV) subissant sous reflux une réaction de transposition qui conduit au composé de formule (V) : 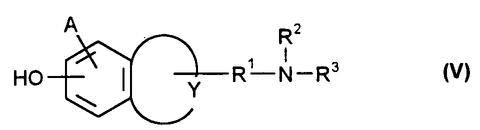 dans laquelle A, R¹, R², R³ et Y sont tels que définis précédemment, ce composé de formule (V) étant ensuite alkylé par un radical de formule R avec R tel que défini dans la formule (I), pour obtenir un composé de formule (I) : 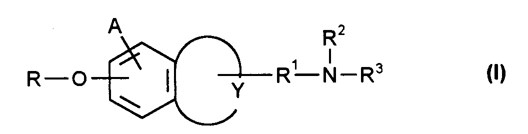 dans laquelle A, R, R¹, R², R³ et Y sont tels que définis précédemment,
composés de formule (I) pouvant être, si on le désire,
- purifiés suivant une ou plusieurs méthodes de purification choisies parmi la cristallisation, la chromatographie sur gel de silice, l'extraction, la filtration, et le passage sur charbon ou résine,
- séparés, le cas échéant, sous forme pure ou sous forme de mélange, en leurs éventuels énantiomères ou diastéréoisomères,
- ou salifiés par une base pharmaceutiquement acceptable.

L'alkylation des composés de formule (V) par un groupement R peut se faire par exemple grâce à un composé de formule (III/b) :

R―Z' (III/b)

où R a la même définition que dans la formule (I) et Z' représente un groupement partant, par exemple un atome d'halogène ou un groupement tosyle, ou grâce à un dialkylsulfate.

L'invention concerne également un procédé de préparation des composés de formule (I/a), cas particulier des composés de formule (I) : 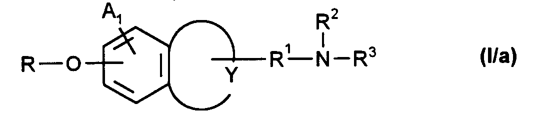 dans laquelle Y, R, R¹, R², R³ sont tels que définis dans la formule (I) et A₁ est un radical alkyle,
caractérisé en ce qu'un composé de formule (I/b) : 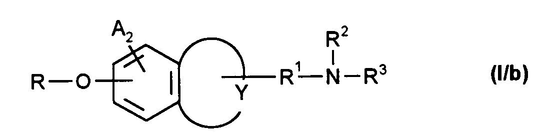 dans laquelle Y, R, R¹, R² et R³ sont tels que précédemment et A₂ est un radical alcényle, est hydrogéné.

Les composés de formule (I) dans lesquels Y forme avec le noyau benzo auquel il est lié un groupement di- ou tétrahydronaphtalène peuvent être obtenus par hydrogénation des composés correspondants de formule (I) dans lesquels Y forme avec le noyau benzo auquel il est lié un groupement naphtalène.

L'invention concerne également un procédé de préparation des composés de formule (I) caractérisé en ce qu'on fait réagir un composé de formule (VI) : 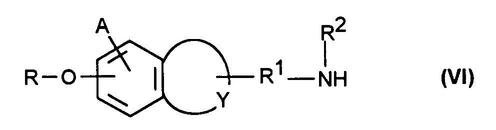 dans laquelle A, R, R¹, R² et Y sont tels que définis dans la formule (I),
a) avec un chlorure d'acyle de formule (VII) : 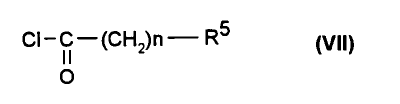 dans laquelle n et R⁵ sont tels que définis dans la formule (I), ou avec l'anhydride d'acide (mixte ou symétrique) correspondant,
b) ou bien avec un iso(thio)cyanate de formule (VIII) :

   X=C=N―CH₂)m―R⁶ (VIII)

   avec X, m et R⁶ tels que définis dans la formule (I) afin d'obtenir, respectivement :
   a) le composé de formule (I/c) : 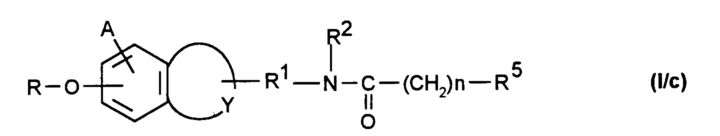 dans laquelle A, R, Y, R¹, R², R⁵ et n sont tels que définis précédemment,
   ou b) le composé de formule (I/d) : 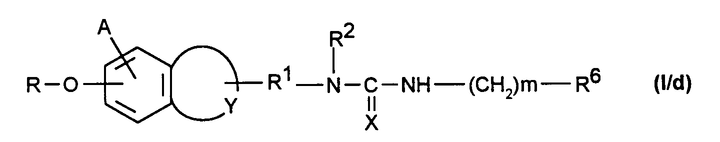 dans laquelle A, R, Y, R¹, R², R⁶, X et m sont tels que définis précédemment, le composé de formule (I/c) pouvant être soumis à un agent de thionation, comme le réactif de Lawesson, pour obtenir les composés de formule (I/e) : 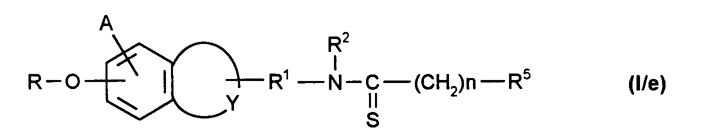 dans laquelle A, R, Y, R¹, R², R⁵ et n sont tels que définis précédemment,
      les composés de formule (I/c), (I/d), et (I/e) formant l'ensemble des composés de formule (I),
      les composés de formule (I) pouvant être, si on le désire,
      - purifiés suivant une ou plusieurs méthodes de purification choisies parmi la cristallisation, la chromatographie sur gel de silice, l'extraction, la filtration, et le passage sur charbon ou résine,
      - séparés, le cas échéant, sous forme pure ou sous forme de mélange, en leurs éventuels énantiomères ou diastéréoisomères,
      - ou salifiés par une base pharmaceutiquement acceptable.

Les matières premières utilisées dans les procédés précédemment décrits sont soit commerciales ou connues dans l'état de la technique, soit aisément accessibles à l'homme du métier selon des procédés bien connus dans la littérature. On se référera plus particulièrement, pour les composés de formule générale (Il), aux descriptions du brevet EP 447 285 et de la demande de brevet EP 530 087, incorporés par référence.

Les composés de formule (I) possèdent des propriétés pharmacologiques très intéressantes pour le clinicien.

Les composés de l'invention et les compositions pharmaceutiques les contenant s'avèrent être utiles pour le traitement des troubles du système mélatoninergique et des troubles liés au système mélatoninergique.

L'étude pharmacologique des dérivés de l'invention a en effet montré qu'ils n'étaient pas toxiques, doués d'une très haute affinité sélective pour les récepteurs de la mélatonine et possédaient d'importantes activités sur le système nerveux central et en particulier, on a relevé des propriétés thérapeutiques sur les troubles du sommeil, des propriétés anxiolytiques, antipsychotiques, analgésiques ainsi que sur la microcirculation qui permettent d'établir que les produits de l'invention sont utiles dans le traitement du stress, des troubles du sommeil, de l'anxiété, des dépressions saisonnières, des pathologies cardiovasculaires, des insomnies et fatigues dues aux décalages horaires, de la schizophrénie, des attaques de panique, de la mélancolie, des troubles de l'appétit, de l'obésité, du psoriasis, des troubles psychotiques, de l'épilepsie, de la maladie de Parkinson, de la démence sénile, des divers désordres liés au vieillissement normal ou pathologique, de la migraine, des pertes de mémoire, de la maladie d'Alzheimer, ainsi que les troubles de la circulation cérébrale. Dans un autre domaine d'activité, il apparaît que les produits de l'invention possèdent des propriétés d'inhibiteurs de l'ovulation, d'immunomodulateurs et qu'ils sont susceptibles d'être utilisés dans le traitement anticancéreux.

Les composés seront utilisés de préférence dans les traitements des dépressions saisonnières, des troubles du sommeil, des pathologies cardiovasculaires, des insomnies et fatigues dues aux décalages horaires, des troubles de l'appétit et de l'obésité.

Par exemple, les composés seront utilisés dans le traitement des dépressions saisonnières et des troubles du sommeil.

La présente invention a également pour objet les compositions pharmaceutiques contenant les produits de formule (I) ou le cas échéant un de leurs sels d'addition à une base pharmaceutiquement acceptable en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, perou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques et les ampoules buvables ou injectables.

La posologie varie selon l'âge et le poids du patient, la voie d'administration, la nature de l'indication thérapeutique, ou des traitements éventuellement associés et s'échelonne entre 0,1 mg et 1 g par 24 heures en 1 ou 2 prises, plus particulièrement 1 à 100 mg, par exemple 1 à 10 mg.

Les exemples suivants illustrent l'invention, mais ne la limitent en aucune façon.

### PREPARATION 1 : N-[2-(7-HYDROXY-NAPHT-1 -YL)ETHYL]ACETAMIDE

### Stade A : BROMHYDRATE DE 2-(7-HYDROXYNAPNT-1-YL)ETHYLAMINE

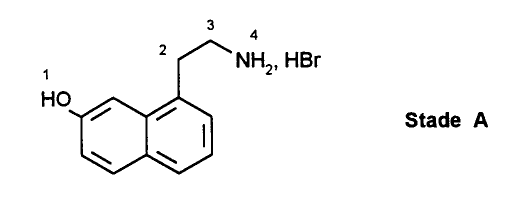

### Réactifs :

| | |
|---|---|
| Chlorhydrate de 2-(7-méthoxy napht-1-yl)éthylamine | 58 mmol (13,8 g) |
| Solution aqueuse de HBr à 47 % | 390 mmol (46 cm³) |

### Mode opératoire :

Dans un ballon de 250 cm³, on introduit le chlorhydrate d'éthylamine et la solution de HBr à 47 %. Le mélange est porté à reflux pendant 5 heures. Après refroidissement, le milieu réactionnel est filtré.

### Caractéristiques :

Masse moléculaire : 268,16 g pour C₁₂H₁₄BrNO
aspect: solide blanc
Point de fusion : 174-175°C
Rf: 0,72 éluant : Méthanol-ammoniaque à 28 % (4-1)
Rendement : 80 %
Solvant de recristallisation : acétate d'éthyle/hexane (1-3)

### Analyse spectroscopique dans l'infra-rouge :

| | | |
|---|---|---|
| 3240-3460 | cm⁻¹ | ν OH |
| 3040-3100 | cm⁻¹ | ν C=C torsion |
| 2950-3060 | cm⁻¹ | ν CH |
| 2480-2720 | cm⁻¹ | ν NH₃+ |

### Analyse spectroscopique de RMN (80 MHz, DMSO-d6 δ) :

| | | | | |
|---|---|---|---|---|
| 3,0-3,4 | ppm | massif | 4H | H2, H3 |
| 7,0-7,9 | ppm | massif | 6H | H aromatiques |
| 8,1 | ppm | singulet | 3H | H4 |
| 9,8 | ppm | singulet | 1H | H1 |

### Analyse élémentaire :

| | % C | % H | % N |
|---|---|---|---|
| Calculé : | 53,75 | 5,26 | 5,22 |
| Trouvé : | 53,84 | 5,30 | 5,32 |

### Stade B : N-[2-(7-HYDROXY NAPHT-1-YL) ETHYL]ACETAMIDE

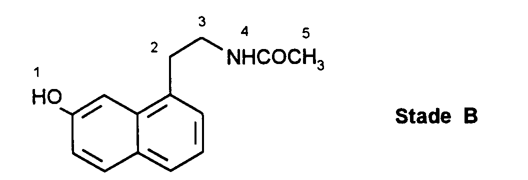

### Réactifs :

| | |
|---|---|
| Bromhydrate de la 2-(7-hydroxy napht-1-yl)éthylamine | 3,8 mmol (1,02 g) |
| Carbonate de sodium | 8,5 mmol (0,90 g) |
| Chlorure d'acétyle | 3,8 mmol (0,30 g) |

### Mode opératoire :

Dans une fiole de 50 cm³, dissoudre dans 5 cm³ d'eau le carbonate de sodium et sous agitation, ajouter le bromhydrate. Ajouter 20 cm³ d'acétate d'éthyle à la suspension obtenue, puis verser goutte à goutte le chlorure d'acétyle. Maintenir l'agitation pendant 30 minutes (la solution est limpide). Extraire la phase organique par de l'eau, puis par une solution aqueuse de HCl 1N, puis par de l'eau jusqu'à neutralité des eaux de lavage. Sécher la phase organique sur du sulfate de magnésium, la filtrer et sécher sous vide réduit.

### Caractéristiques :

Masse moléculaire : 229,27 g pour C₁₄H₁₅NO₂
aspect : solide blanc
Point de fusion : 125-126°C
Rf : 0,32 éluant : Acétone-Toluène-Cyclohexane (4-4-2)
Rendement : 60 %
Solvant de recristallisation : eau

### Analyse spectroscopique dans l'infrarouge :

| | | |
|---|---|---|
| 3340 | cm⁻¹ | ν OH |
| 2980 | cm⁻¹ | ν CH |
| 1640 | cm⁻¹ | ν CO amide |

### Analyse spectroscopique de RMN (80 MHz, CDCl₃, δ) :

| | | | | |
|---|---|---|---|---|
| 2,0 | ppm | singulet | 3H | H5 |
| 3,2 | ppm | triplet | 2H | H2 J₂₋₃ = 7,1 Hz |
| 3,6 | ppm | multiplet | 2H | H3 |
| 5,8 | ppm | signal | 1H | H4 |
| 7,0-7,9 | ppm | massif | 6H | H aromatiques |
| 9,8 | ppm | singulet | 1H | H1 |

### Analyse élémentaire :

| | % C | % H | % N |
|---|---|---|---|
| Calculé : | 73,34 | 6,59 | 6,11 |
| Trouvé : | 72,99 | 6,57 | 6,29 |

### PREPARATIONS 2 A 10 :

En procédant de façon analogue à celle décrite pour la préparation 1 mais en utilisant soit le chlorure d'acyle ou l'anhydride d'acide approprié, on obtient les préparations suivantes :
- **PREPARATION 2 :**: **N-[2-(7-HYDROXY-NAPHT-1-YL)ETHYL]FORMAMIDE**
- **PREPARATION 3 :**: **N-[2-(7-HYDROXY-NAPHT-1-YL)ETHYL]PROPIONAMIDE**
- **PREPARATION 4 :**: **N-[2-(7-HYDROXY-NAPHT-1-YL)ETHYL]BUTYRAMIDE**
- **PREPARATION 5 :**: **N-[2-(7-HYDROXY-NAPHT-1-YL)ETHYL]PENTANAMIDE**
- **PREPARATION 6 :**: **N-[2-(7-HYDROXY-NAPHT-1-YL)ETHYL]HEXANAMIDE**
- **PREPARATION 7 :**: **N-[2-(7-HYDROXY-NAPHT-1-YL)ETHYL]HEPTANAMIDE**
- **PREPARATION 8 :**: **N-[2-(7-HYDROXY-NAPHT-1-YL)ETHYL]CYCLOPROPANE-CARBOXAMIDE**
- **PREPARATION 9 :**: **N-[2-(7-HYDROXY-NAPHT-1-YL)ETHYL]CYCLOBUTANE-CARBOXAMIDE**
- **PREPARATION 10 :**: **N-[2-(7-HYDROXY-NAPHT-1-YL)ETHYL]TRIFLUOROACETAMIDE**
- **PREPARATION 11 :**: **N-[2-(7-HYDROXY-NAPHT-1-YL)ETHYL]IODOACETAMIDE**

### PREPARATION 12 : N-[2-(7-HYDROXY-NAPHT-1-YL)ETHYL]N'-METHYLUREE

En faisant réagir le chlorhydrate de 2-(7-méthoxy napht-1-yl)éthylamine avec l'isocyanate de méthyle, on obtient le composé du titre.

### PREPARATIONS 13 A 30 :

En procédant comme dans la préparation 12 mais en utilisant l'iso(thio)cyanate approprié, on obtient les composés suivants :
- **PREPARATION 13 :**: **N-[2-(7-HYDROXY-NAPHT-1-YL)ETHYL]UREE**
- **PREPARATION 14 :**: **N-[2-(7-HYDROXY-NAPHT-1-YL)ETHYL]-N'-ETHYLUREE**
- **PREPARATION 15 :**: **N-[2-(7-HYDROXY-NAPHT-1-YL)ETHYL]-N-'PROPYLUREE**
- **PREPARATION 16 :**: **N-[2-(7-HYDROXY-NAPHT-1-YL)ETHYL]-N'-BUTYLUREE**
- **PREPARATION 17 :**: **N-[2-(7-HYDROXY-NAPHT-1-YL)ETHYL]-N'-PENTYLUREE**
- **PREPARATION 18 :**: **N-[2-(7-HYDROXY-NAPHT-1-YL)ETHYL]-N'-HEXYLUREE**
- **PREPARATION 19 :**: **N-[2-(7-HYDROXY-NAPHT-1-YL)ETHYL]-N'-CYCLOPROPYLUREE**
- **PREPARATION 20 :**: **N-[2-(7-HYDROXY-NAPHT-1-YL)ETHYL]-N'-CYCLOBUTYLUREE**
- **PREPARATION 21 :**: **N-[2-(7-HYDROXY-NAPHT-1-YL)ETHYL]-N'-CYCLOHEXYLUREE**
- **PREPARATION 22 :**: **N-[2-(7-HYDROXY-NAPHT-1-YL)ETHYL]THIOUREE**
- **PREPARATION 23 :**: **N-[2-(7-HYDROXY-NAPHT-1-YL)ETHYL]-N'-METHYLTHIOUREE**
- **PREPARATION 24 :**: **N-[2-(7-HYDROXY-NAPHT-1-YL)ETHYL]-N'-ETHYLTHIOUREE**
- **PREPARATION 25 :**: **N-[2-(7-HYDROXY-NAPHT-1-YL)ETHYL]-N'-PROPYLTHIOUREE**
- **PREPARATION 26 :**: **N-[2-(7-HYDROXY-NAPHT-1-YL)ETHYL]-N'-BUTYLTHIOUREE**
- **PREPARATION 27 :**: **N-[2-(7-HYDROXY-NAPHT-1-YL)ETHYL]-N'-PENTYLTHIOUREE**
- **PREPARATION 28 :**: **N-[2-(7-HYDROXY-NAPHT-1-YL)ETHYL]-N'-HEXYLTHIOUREE**
- **PREPARATION 29 :**: **N-[2-(7-HYDROXY-NAPHT-1-YL)ETHYL]-N'-ISOPROPYLTHIOUREE**
- **PREPARATION 30 :**: **N-[2-(7-HYDROXY-NAPHT-1-YL)ETHYL]N'-ISOBUTYLTHIOUREE**
- **PREPARATION 31 :**: **N-[2-(5-HYDROXY-INDOL-3-YL)ETHYL]ACETAMIDE**
(J. Med. Chem. 1994, 37, pp 2828-2830)
- **PREPARATION 32 :**: **N-[2-(5-HYDROXY-BENZOFUR-3-YL)ETHYL]ACETAMIDE**
- **PREPARATION 33 :**: **N-[2-(5-HYDROXY-BENZOTHIOPHEN-3-YL)ETHYL]ACETAMIDE**
(J. Med. Chem. 1970, 13, pp 1205-1208)

### EXEMPLE 1 : N-[2-(7-METHOXY 8-ALLYL NAPHT-1-YL)ETHYL]ACETAMIDE

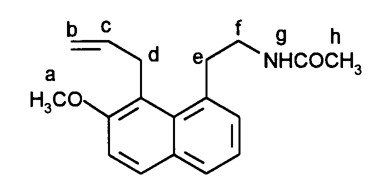

### Stade A : N-[2-(7-ALLYLOXY NAPHT-1-YL) ETHYL]ACETAMIDE

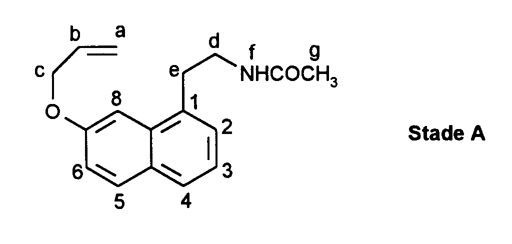

### Réactifs :

| | |
|---|---|
| N-[2-(7-hydroxy napht-1-yl) éthyl]acétamide | 20 mmol (5 g) |
| Carbonate de potassium | 50 mmol (6,91 g) |
| Bromure d'allyle | 30 mmol (3,63 g) |

### Mode opératoire :

Dissoudre le composé obtenu à la préparation 1 dans 100 cm³ d'acétone anhydre. Ajouter le carbonate de potassium et laisser sous agitation à reflux pendant 30 minutes. Ajouter goutte à goutte le bromure d'allyle. Laisser à reflux et sous agitation pendant 3 heures. Après refroidissement, filtrer le milieu réactionnel et sécher sous vide réduit le filtrat. L'huile obtenue est purifiée par chromatographie sur colonne.

### Caractéristiques :

Masse moléculaire : 269,33 g pour C₁₇H₁₉NO₂
aspect : huile
Rf : 0,190 éluant : Acétone-Toluène-Cyclohexane (2-3-5)
Rendement : 87 %

### Analyse spectroscopique dans l'infra-rouge :

| | | |
|---|---|---|
| 3260 | cm⁻¹ | ν NH amide |
| 2920-2840 | cm⁻¹ | ν CH |
| 1635 | cm⁻¹ | ν CO amide |
| 1590 | cm⁻¹ | ν C=C |

### Analyse spectroscopique de RMN (300 MHz, CDCl₃, δ) :

| | | | | | |
|---|---|---|---|---|---|
| 1,90 | ppm | singulet | 3H | Hg | |
| 3,20 | ppm | triplet | 2H | He | J_{e-d} = 7,0 Hz |
| 3,60 | ppm | multiplet | 2H | Hd | |
| 4,70 | ppm | doublet | 2H | Hc | J_{c-b} = 5,3 Hz |
| 5,30 | ppm | doublet | 1H | Ha cis | J_{a-b} = 10,5 Hz |
| 5,50 | ppm | doublet | 1H | Ha trans | J_{a-b} = 17,3 Hz |
| 5,60 | ppm | signal | 1H | Hf | |
| 6,15 | ppm | multiplet | 1H | Hb | |
| 7,15 | ppm | doublet dédoublé | 1H | H6 | Jₒᵣₜₕₒ=8,9 Hz ; J_{méta}=2,3 Hz |
| 7,18-7,22 | ppm | massif | 2H | H2,3 | |
| 7,40 | ppm | doublet | 1H | H8 | J_{méta}=2,3Hz |
| 7,65 | ppm | multiplet | 1H | H4 | |
| 7,75 | ppm | doublet | 1H | H5 | Jₒᵣₜₕₒ=8,9 Hz |

### Analyse élémentaire :

| | % C | % H | % N |
|---|---|---|---|
| Calculé : | 75,80 | 7,11 | 5,20 |
| Trouvé : | 75,75 | 7,15 | 5,20 |

### Stade B : N-[2-(7-HYDROXY 8-ALLYL NAPHT-1-YL) ETHYL]ACETAMIDE

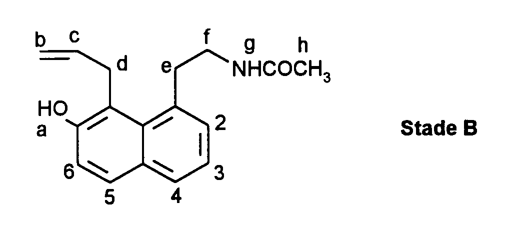

### Réactifs :

| | |
|---|---|
| N-[2-(7-allyloxy napht-1-yl) éthyl]acétamide | 7,4 mmol (2 g) |
| N,N-diméthylaniline | 7,4 mmol (10 cm³) |

### Mode opératoire :

Dissoudre le N-[2-(7-allyloxy napht-1-yl)éthyl]acétamide dans la N,N-diméthylaniline, porter le milieu réactionnel à reflux (200°C) pendant 2 heures. Après refroidissement, ajouter 20 cm³ d'éther et extraire la phase organique par une solution aqueuse de soude 10 % puis par de l'eau. La phase aqueuse est ensuite acidifiée par une solution aqueuse HCl 6N et laissée sous agitation pendant quelques minutes. Filtrer le précipité obtenu.

### Caractéristiques :

Masse moléculaire : 269,33 g pour C₁₇H₁₉NO₂
aspect : solide jaune pâle
Température de fusion : 157-159°C
Rf : 0,38 éluant : Acétone-Toluène-Cyclohexane (4-4-2)
Rendement : 84 %
Solvant de recristallisation : cyclohexane

### Analyse spectroscopique dans l'infra-rouge :

| | | |
|---|---|---|
| 3280 | cm⁻¹ | ν NH amide |
| 2860-3000 | cm⁻¹ | ν CH |
| 1600 | cm⁻¹ | ν CO amide |

### Analyse spectroscopique de RMN (300 MHz,DMSO-d6, δ) :

| | | | | | |
|---|---|---|---|---|---|
| 1,83 | ppm | singulet | 3H | Hh | |
| 3,20 | ppm | signal | 2H | He | |
| 3,25 | ppm | signal | 2H | Hf | |
| 3,90 | ppm | signal | 2H | Hd | |
| 4,65 | ppm | doublet | 1H | Hb trans | J_{b-c} = 17,2 Hz |
| 4,95 | ppm | doublet | 1H | Hb cis | J_{b-c} = 8,8 Hz |
| 6,05 | ppm | multiplet | 1H | Hc | |
| 7,14-7,23 | ppm | massif | 3H | H2,3,6 | |
| 7,64-7,68 | ppm | massif | 2H | H4,5 | |
| 8,08 | ppm | signal | 1H | Hg | |
| 9,60 | ppm | singulet | 1H | Ha échangeable dans D₂O | |

### Analyse élémentaire :

| | % C | % H | % N |
|---|---|---|---|
| Calculé : | 75,81 | 7,11 | 5,20 |
| Trouvé : | 75,72 | 7,09 | 5,31 |

### Stade C : N-[2-(7-METHOXY 8-ALLYL NAPHT-1-YL) ETHYL]ACETAMIDE

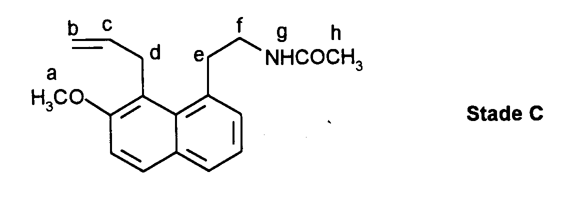

### Réactifs :

| | |
|---|---|
| N-[2-(7-hydroxy 8-allyl napht-1-yl) éthyl]acétamide | (1g) 4.10⁻³ mole |
| Solution aqueuse de soude à 10 % | (32 cm³) 0,08 mode |
| Diméthylsulfate | (7,6 cm³) 0,08 mole |

### Mode opératoire :

Dissoudre le N-[2-(7-hydroxy 8-allyl napht-1-yl)éthyl]acétamide dans la solution aqueuse de soude à 50°C. Arrêter le chauffage au bout de 10 minutes et laisser refroidir sous agitation, puis ajouter goutte à goutte le diméthylsulfate. Extraire la phase aqueuse par 3 fois 20 cm³ d'éther, laver la phase organique par une solution aqueuse de carbonate de potassium à 10 % puis par de l'eau jusqu'à neutralité des eaux de lavage. Sécher la phase organique sur MgSO₄, la filtrer et l'évaporer sous vide.

### Caractéristiques :

283,37g/mode pour C₁₈H₂₁NO₂
aspect : solide blanchâtre
Température de fusion : 98-99°C Rf : 0,38 éluant : Acétone-Toluène-Cyclohexane (4-4-2)
Rendement : 81 %
Solvant de recristallisation : toluène- cyclohexane (1-5)

### Analyse spectroscopique dans l'infrarouge :

| | | |
|---|---|---|
| 3260 et 3060 | cm⁻¹ | ν NH amide |
| 3000-2820 | cm⁻¹ | ν CH |
| 1630 | cm⁻¹ | ν CO amide |
| 1610 et 1590 | cm⁻¹ | ν C=C |
| 1250 | cm⁻¹ | ν CH₃O |

### Analyse spectroscopique de RMN (300 MHz, CDCl₃, δ) :

| | | | | | |
|---|---|---|---|---|---|
| 1,94 | ppm | singulet | 3H | Hh | |
| 3,37 | ppm | triplet | 2H | He | J_{e-f} = 7,1 Hz |
| 3,54 | ppm | multiplet | 2H | Hf | |
| 3,94 | ppm | singulet | 3H | Ha | |
| 3,98 | ppm | doublet | 2H | Hd | J_{d-c} = 4,6 Hz |
| 4,73 | ppm | doublet | 1H | Hb trans | J_{b-c} = 17,3 Hz |
| 5,02 | ppm | doublet | 1H | Hb cis | J_{b-c} = 10,2 Hz |
| 5,50 | ppm | signal | 1H | Hg | |
| 6,15 | ppm | multiplet | 1H | Hc | |
| 7,21-7,33 | ppm | massif | 3H | H2,3,6 | |
| 7,69 | ppm | doublet | 1H | H4 | Jₒᵣₜₕₒ = 7,4 Hz |
| 7,79 | ppm | doublet | 1H | H5 | Jₒᵣₜₕₒ = 9,0 Hz |

### Analyse élémentaire :

| | % C | % H | % N |
|---|---|---|---|
| Calculé : | 76,30 | 7,47 | 4,94 |
| Trouvé : | 76,51 | 7,64 | 5,21 |

### EXEMPLES 2 A 14 :

En procédant comme dans l'exemple 1, mais en substituant la fonction hydroxy par le radical approprié, on obtient les composés des exemples suivants :
- **EXEMPLE 2 :**: **N-[2-(7-ETHOXY 8-ALLYL NAPHT-1-YL)ETHYL]ACETAMIDE**
- **EXEMPLE 3 :**: **N-[2-(7-PROPOXY 8-ALLYL NAPHT-1-YL)ETHYL]ACETAMIDE**
point de fusion : 130-131°C
- **EXEMPLE 4 :**: **N-[2-(7-BUTYLOXY 8-ALLYL NAPHT-1-YL)ETHYL]ACETAMIDE**
- **EXEMPLE 5 :**: **N-[2-(7-PENTYLOXY 8-ALLYL NAPHT-1-YL)ETHYL]ACETAMIDE**
- **EXEMPLE 6 :**: **N-[2-(7-HEXYLOXY 8-ALLYL NAPHT-1-YL)ETHYL]ACETAMIDE**
- **EXEMPLE 7 :**: **N-[2-(7-CYCLOPROPYLMETHOXY 8-ALLYL NAPHT-1-YL)ETHYL] ACETAMIDE**
- **EXEMPLE 8 :**: **N-[2-(7-CYCLOHEXYLOXY 8-ALLYL NAPHT-1-YL)ETHYL]ACETAMIDE**
- **EXEMPLE 9 :**: **N-[2-(7-ALLYLOXY 8-ALLYL NAPHT-1-YL)ETHYL]ACETAMIDE**
- **EXEMPLE 10 :**: **N-[2-(7-VINYLOXY 8-ALLYL NAPHT-1-YL)ETHYL]ACETAMIDE**
- **EXEMPLE 11 :**: **N-[2-(7-PROPARGYLOXY 8-ALLYL NAPHT-1-YL)ETHYL]ACETAMIDE**
- **EXEMPLE 12 :**: **N-[2-(7-BENZYLOXY 8-ALLYL NAPHT-1-YL)ETHYL]ACETAMIDE**
- **EXEMPLE 13 :**: **N-[2-(7-BENZHYDRYLOXY 8-ALLYL NAPHT-1-YL)ETHYL]ACETAMIDE**
- **EXEMPLE 14 :**: **N-[2-(7-CYCLOHEXEN-3-YLOXY 8-ALLYL NAPHT-1-YL)ETHYL]-ACETAMIDE**

### EXEMPLES 15 A 46 :

En procédant comme dans l'exemple 1, mais en utilisant les préparations appropriées, on obtient les composés des exemples suivants :
- **EXEMPLE 15 :**: **N-[2-(7-METHOXY 8-ALLYL NAPHT-1-YL)ETHYL]FORMAMIDE**
- **EXEMPLE 16 :**: **N-[2-(7-METHOXY 8-ALLYL NAPHT-1-YL)ETHYL]PROPIONAMIDE**
Point de fusion : 90-92°C
- **EXEMPLE 17 :**: **N-[2-(7-METHOXY 8-ALLYL NAPHT-1-YL)ETHYL]BUTYRAMIDE**
Point de fusion : 75-77°C
- **EXEMPLE 18 :**: **N-[2-(7-METHOXY 8-ALLYL NAPHT-1-YL)ETHYL]PENTANAMIDE**
Point de fusion : 60-62°C
- **EXEMPLE 19 :**: **N-[2-(7-METHOXY 8-ALLYL NAPHT-1-YL)ETHYL]HEXANAMIDE**
- **EXEMPLE 20 :**: **N-[2-(7-METHOXY 8-ALLYL NAPHT-1-YL)ETHYL]HEPTANAMIDE**
- **EXEMPLE 21 :**: **N-[2-(7-METHOXY 8-ALLYL NAPHT-1-YL)ETHYL]CYCLOPROPANE-CARBOXAMIDE**
Point de fusion : 112-114°C
- **EXEMPLE 22 :**: **N-[2-(7-METHOXY 8-ALLYL NAPHT-1-YL)ETHYL]CYCLOBUTANE-CARBOXAMIDE**
Point de fusion : 92-96°C
- **EXEMPLE 23 :**: **N-[2-(7-METHOXY 8-ALLYL NAPHT-1-YL)ETHYL]TRIFLUORO-ACETAMIDE**
- **EXEMPLE 24 :**: **N-[2-(7-METHOXY 8-ALLYL NAPHT-1-YL)ETHYL]IODOACETAMIDE**
- **EXEMPLE 25 :**: **N-[2-(7-METHOXY 8-ALLYL NAPHT-1-YL)ETHYL]-N'-METHYLUREE**
- **EXEMPLE 26 :**: **N-[2-(7-METHOXY 8-ALLYL NAPHT-1-YL)ETHYL]UREE**
- **EXEMPLE 27 :**: **N-[2-(7-METHOXY 8-ALLYL NAPHT-1-YL)ETHYL]-N'-ETHYLUREE**
- **EXEMPLE 28 :**: **N-[2-(7-METHOXY 8-ALLYL NAPHT-1-YL)ETHYL]-N'-PROPYLUREE**
- **EXEMPLE 29 :**: **N-[2-(7-METHOXY 8-ALLYL NAPHT-1-YL)ETHYL]-N'-BUTYLUREE**
- **EXEMPLE 30 :**: **N-[2-(7-METHOXY 8-ALLYL NAPHT-1-YL)ETHYL]-N'-PENTYLUREE**
- **EXEMPLE 31 :**: **N-[2-(7-METHOXY 8-ALLYL NAPHT-1-YL)ETHYL]-N'-HEXYLUREE**
- **EXEMPLE 32 :**: **N-[2-(7-METHOXY 8-ALLYL NAPHT-1-YL)ETHYL]-N'-CYCLOPROPYLUREE**
- **EXEMPLE 33 :**: **N-[2-(7-METHOXY 8-ALLYL NAPHT-1-YL)ETHYL]-N'-CYCLOBUTYLUREE**
- **EXEMPLE 34 :**: **N-[2-(7-METHOXY 8-ALLYL NAPHT-1-YL)ETHYL]-N'-CYCLOHEXYLUREE**
- **EXEMPLE 35 :**: **N-[2-(7-METHOXY 8-ALLYL NAPHT-1-YL)ETHYL]THIOUREE**
- **EXEMPLE 36 :**: **N-[2-(7-METHOXY 8-ALLYL NAPHT-1-YL)ETHYL]-N'-METHYLTHIOUREE**
- **EXEMPLE 37 :**: **N-[2-(7-METHOXY 8-ALLYL NAPHT-1-YL)ETHYL]-N'-ETHYLTHIOUREE**
- **EXEMPLE 38 :**: **N-[2-(7-METHOXY 8-ALLYL NAPHT-1-YL)ETHYL]-N'-PROPYLTHIOUREE**
- **EXEMPLE 39 :**: **N-[2-(7-METHOXY 8-ALLYL NAPHT-1-YL)ETHYL]-N'-BUTYLTHIOUREE**
- **EXEMPLE 40 :**: **N-[2-(7-METHOXY 8-ALLYL NAPHT-1-YL)ETHYL]-N'-PENTYLTHIOUREE**
- **EXEMPLE 41 :**: **N-[2-(7-METHOXY 8-ALLYL NAPHT-1-YL)ETHYL]-N'-HEXYLTHIOUREE**
- **EXEMPLE 42 :**: **N-[2-(7-METHOXY 8-ALLYL NAPHT-1-YL)ETHYL]-N'-ISOPROPYLTHIOUREE**
- **EXEMPLE 43 :**: **N-[2-(7-METHOXY 8-ALLYL NAPHT-1-YL)ETHYL]-N'-ISOBUTYLTHIOUREE**
- **EXEMPLE 44 :**: **N-[2-(5-METHOXY 4-ALLYL INDOL-3-YL)ETHYL]ACETAMIDE**
- **EXEMPLE 45 :**: **N-[2-(5-METHOXY 4-ALLYL BENZOFUR-3-YL)ETHYL]ACETAMIDE**
- **EXEMPLE 46 :**: **N-[2-(5-METHOXY 4-ALLYL BENZOTHIOPHEN-3-YL)ETHYL]-ACETAMIDE**

### EXEMPLES 47 A 54 :

En procédant comme dans l'exemple 1, mais en utilisant les réactifs de formule (III/a) appropriés, on obtient les composés des exemples suivants :
- **EXEMPLE 47 :**: **N-[2-(7-METHOXY 8-VINYL NAPHT-1-YL)ETHYL]ACETAMIDE**
- **EXEMPLE 48 :**: **N-[2-(7-METHOXY 8-(PROP-1-EN-1-YL) NAPHT-1-YL)ETHYL]-ACETAMIDE**
- **EXEMPLE 49 :**: **N-[2-(7-METHOXY 8-ISOPROPENYL NAPHT-1-YL)ETHYL]-ACETAMIDE**
- **EXEMPLE 50 :**: **N-[2-(7-METHOXY 8-(BUT-2-EN-1-YL) NAPHT-1-YL)ETHYL]ACETAMIDE**
- **EXEMPLE 51 :**: **N-[2-(7-METHOXY 8-(BUT-3-EN-1-YL)NAPHT-1-YL)ETHYL]ACETAMIDE**
- **EXEMPLE 52 :**: **N-[2-(7-METHOXY 8-(PENT-4-EN-1-YL) NAPHT-1-YL)ETHYL]ACETAMIDE**
- **EXEMPLE 53 :**: **N-[2-(7-METHOXY 8-(HEX-5-EN-1-YL) NAPHT-1-YL)ETHYL]ACETAMIDE**
- **EXEMPLE 54 :**: **N-[2-(7-METHOXY 8-PROPARGYL NAPHT-1-YL)ETHYL]ACETAMIDE**
- **EXEMPLE 55 :**: **N-[2-(7-METHOXY 8-PROPYL NAPHT-1-YL)ETHYL]ACETAMIDE**

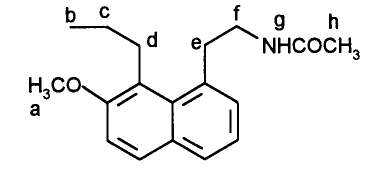

### Réactifs :

| | |
|---|---|
| N-[2-(7-méthoxy 8-allyl napht-1-yl)éthyl]acétamide | 0,8 g (2,8.10⁻³ mole) |
| Hydrazine à 98 % | 0,41 cm³ (8,4.10⁻³ mole) |
| Sulfate de cuivre | 0,05 g (2,8.10⁻⁴ mole) |
| Ethanol 95° | 20 cm³ |

### Mode opératoire :

Dans un bicol de 100 cm³, introduire le N-[2-(7-méthoxy 8-allyl napht-1-yl)éthyl]acétamide (exemple 1) dans 20 cm³ d'éthanol et faire buller de l'air (contenant O₂) dans le milieu réactionnel. Ajouter l'hydrazine et le sulfate de cuivre sous agitation et sous air. Laisser sous agitation pendant 48 heures. Filtrer et évaporer le filtrat. Reprendre par 20 cm³ d'éther et laver la phase organique par une solution aqueuse HCl 2N puis par de l'eau jusqu'à neutralité des eaux de lavage. Sécher sur CaCl₂, filtrer et évaporer.

### Caractéristiques :

285,39 g/mole pour C₁₈H₂₃NO₂
aspect : solide blanc
Température de fusion : 103-105°C
Rf : 0,39 éluant : Acétone-Toluène-Cyclohexane (4-4-2)
Rendement : 45 %
Solvant de recristallisation : alcool-eau (3-1)

### Analyse spectroscopique dans l'Infrarouge :

| | | |
|---|---|---|
| 3300 et 3030 | cm⁻¹ | ν NH amide |
| 3000-2820 | cm⁻¹ | ν CH |
| 1660 | cm⁻¹ | ν CO amide |
| 1600 et 1590 | cm⁻¹ | ν C=C |
| 1250 | cm⁻¹ | ν CH₃O |

### Analyse spectroscopique de RMN (80 MHz, CDCl₃, δ) :

| | | | | | |
|---|---|---|---|---|---|
| 1,00 | ppm | triplet | 3H | Hb | J_{b-c} = 6,80Hz |
| 1,60 | ppm | multiplet | 2H | Hc | |
| 1,95 | ppm | singulet | 3H | Hh | |
| 3,00-3,65 | ppm | massif | 6H | He,d,f | |
| 3,95 | ppm | singulet | 3H | Ha | |
| 5,40 | ppm | signal | 1H | Hg | |
| 7,20-7,35 | ppm | massif | 3H | H2,3,6 | |
| 7,60-7,80 | ppm | massif | 2H | H4,5 | |

### Analyse élémentaire :

| | % C | % H | % N |
|---|---|---|---|
| Calculé : | 75,76 | 8,12 | 4,91 |
| Trouvé : | 75,72 | 8,02 | 4,91 |

### EXEMPLES 56 A 60:

En procédant comme dans l'exemple 55, mais en partant des composés des exemples appropriés, on obtient les composés des exemples suivants :
- **EXEMPLE 56 :**: **N-[2-(7-METHOXY 8-ETHYL NAPHT-1-YL)ETHYL]ACETAMIDE**
- **EXEMPLE 57 :**: **N-[2-(7-METHOXY 8-ISOPROPYL NAPHT-1-YL)ETHYL]ACETAMIDE**
- **EXEMPLE 58 :**: **N-[2-(7-METHOXY 8-BUTYL NAPHT-1-YL)ETHYL]ACETAMIDE**
- **EXEMPLE 59 :**: **N-[2-(7-METHOXY 8-PENTYL NAPHT-1-YL)ETHYL]ACETAMIDE**
- **EXEMPLE 60 :**: **N-[2-(7-METHOXY 8-HEXYL NAPHT-1-YL)ETHYL]ACETAMIDE**
- **EXEMPLE 61 :**: **N-[2-(7-METHOXY 8-PROPYL 1,2,3,4-TETRAHYDRONAPHT-1-YL) ETHYL]ACETAMIDE**

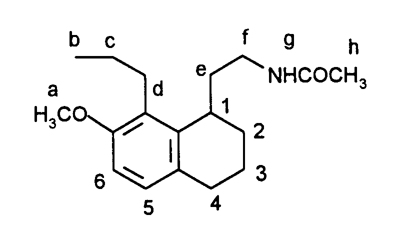

### Réactifs :

| | |
|---|---|
| N-[2-(7-méthoxy 8-allyl napht-1-yl) éthyl]acétamide | 1 g (3,5. 10⁻³ mole) |
| Pd/C | 1 g |
| Ethanol 95° | 20 cm³ |

### Mode opératoire :

Dissoudre le N-[2-(7-méthoxy 8-allyl napht-1-yl)éthyl]acétamide (exemple 1) dans l'éthanol, ajouter le charbon palladié (Pd/C) et mettre le milieu réactionnel sous agitation sous H₂ à pression atmosphérique et température ambiante. La réaction est terminée au bout de 3 heures, le milieu est filtré et le filtrat est évaporé. Recristalliser le solide obtenu.

### Caractéristiques :

289,42 g/mole pour C₁₈H₂₇NO₂
aspect: solide blanc
Température de fusion : 110-112°C
Rf : 0,15 éluant : Acétone-Toluène-Cyclohexane (2-3-5)
Rendement : 87 %
Solvant de recristallisation : alcool-eau (1-5)

### Analyse spectroscopique dans l'Infrarouge :

| | | |
|---|---|---|
| 3260 et 3080 | cm⁻¹ | ν NH amide |
| 3000-2820 | cm⁻¹ | ν CH |
| 1640 | cm⁻¹ | ν CO amide |
| 1250 | cm⁻¹ | ν CH₃O |

### Analyse spectroscopique de RMN (80 MHz, CDCl₃, δ) :

| | | | | | |
|---|---|---|---|---|---|
| 1,00 | ppm | triplet | 3H | Hb | J_{b-c} = 7,30 Hz |
| 1,20-1,90 | ppm | massif | 8H | Hc,e,2 et 3 | |
| 2,00 | ppm | singulet | 3H | Hh | |
| 2,20-2,60 | ppm | massif | 1H | H1 | |
| 2,60-3,10 | ppm | massif | 4H | Hd,4 | |
| 3,20-3,55 | ppm | massif | 2H | Hf | |
| 3,80 | ppm | singulet | 3H | Ha | |
| 5,45 | ppm | signal | 1H | Hg | |
| 6,65 | ppm | doublet | 1H | H6 | J₆₋₅ = 8,70 Hz |
| 6,90 | ppm | doublet | 3H | H5 | J₅₋₆ = 8,70 Hz |

### Analyse élémentaire :

| | % C | % H | % N |
|---|---|---|---|
| Calculé : | 74,70 | 9,40 | 4,84 |
| Trouvé : | 74,76 | 9,68 | 4,97 |

### ETUDE PHARMACOLOGIQUE

### EXEMPLE A : ETUDE DE LA TOXICITE AIGUE

La toxicité aiguë a été appréciée après administration orale à des lots de 8 souris (26 ± 2 grammes). Les animaux ont été observés à intervalles réguliers au cours de la première joumée et quotidiennement pendant les deux semaines suivant le traitement. La DL 50, entraînant la mort de 50 % des animaux, a été évaluée.

La DL 50 des produits testés est supérieure à 1000 mg.kg⁻¹ ce qui indique la faible toxicité des composés de l'invention.

### EXEMPLE B : ETUDE DE LIAISON AUX RECEPTEURS DE LA MELATONINE

### B1) ETUDE SUR DES CELLULES DE LA PARS TUBERALIS DE MOUTON

Les études de liaison aux récepteurs de la mélatonine des composés de l'invention ont été réalisées selon les techniques classiques sur les cellules de la pars tuberalis de mouton. La pars tuberalis de l'adénohypophyse est en effet caractérisée, chez les mammifères, par une haute densité en récepteurs de la mélatonine (Joumal of Neuroendocrinology vol. (1), pp 1-4 (1989)).

### PROTOCOLE

1) Les membranes de pars Tuberalis de mouton sont préparées et utilisées comme tissu cible dans des expériences de saturation pour déterminer les capacités et affinités de liaison pour la 2-[¹²⁵I]iodomélatonine.
2) Les membranes de Pars tuberalis de mouton sont utilisées comme tissu cible, avec les différents composés à tester, dans des expériences de liaison compétitive par rapport à la 2-iodomélatonine.

Chaque expérience est réalisée en triple et une gamme de concentrations différentes est testée pour chaque composé.

Les résultats permettent de déterminer, après traitement statistique, les affinités de liaison du composé testé.

### RESULTATS

Il apparaît que les composés de l'invention possèdent une puissante affinité pour les récepteurs de la mélatonine puisqu'ils se fixent avec une constante de dissociation de l'ordre de 10⁻¹¹ M.

### B2) ETUDE SUR DES MEMBRANES DE CELLULES DU CERVEAU DE POULET (GALLUS DOMESTICUS)

Les animaux utilisés sont des poulets (Gallus domesticus) agés de 12 jours. Ils sont sacrifiés entre 13 et 17 heures le jour de leur arrivée. Les cerveaux sont rapidement prélevés et congelés à - 200°C puis conservés à - 80°C. Les membranes sont préparées selon la méthode décrite par Yuan et Pang (Journal of Endocrinology 128, pages 475-482, 1991). La 2-[¹²⁵I]iodomélatonine est incubée en présence des membranes dans une solution tamponnée à pH 7.4 pendant 60 min à 25°C. A l'issue de cette période, la suspension membranaire est filtrée (Whatman GF/C). La radioactivité retenue sur le filtre est déterminée à l'aide d'un compteur à scintillation liquide Beckman® LS 6000.

Les produits utilisés sont :
- 2-[¹²⁵I]iodomélatonine
- mélatonine
- produits courants
- molécules originales

En screening primaire, les molécules sont testées à 2 concentrations (10⁻⁷ et 10⁻⁵ M). Chaque résultat est la moyenne de 3 mesures indépendantes. Les molécules actives retenues d'après les résultats du screening primaire ont fait l'objet d'une détermination quantitative de leur efficacité (IC₅₀). Elles sont utilisées à 10 concentrations différentes.

Les composés de l'invention présentent une très forte affinité pour les récepteurs de la mélatonine.

### EXEMPLE C : TEST DES QUATRE PLAQUES

Les produits de l'invention sont administrés par voie oesophagienne à des lots de dix souris. Un lot reçoit du sirop de gomme. 30 minutes après l'administration des produits à étudier, les animaux sont placés dans des habitacles dont le plancher comprend quatre plaques métalliques. Chaque fois que l'animal passe d'une plaque à l'autre, il reçoit une légère décharge électrique (0,35 mA). Le nombre de passages est enregistré pendant une minute. Après administration, les composés de l'invention augmentent de façon significative le nombre de passages ce qui montre l'activité anxiolytique des dérivés de l'invention.

### EXEMPLE D: COMPOSES DE L'INVENTION SUR LES RYTHMES CIRCADIENS D'ACTIVITE LOCOMOTRICE DU RAT

L'implication de la mélatonine dans l'entraînement, par l'altemance jour/nuit, de la plupart des rythmes circadiens physiologiques, biochimiques et comportementaux a permis d'établir un modèle pharmacologique pour la recherche de ligands mélatoninergiques.

Les effets des molécules sont testés sur de nombreux paramètres et en particulier sur les rythmes circadiens d'activité locomotrice qui représentent un marqueur fiable de l'activité de l'horloge circadienne endogène.

Dans cette étude, on évalue les effets de telles molécules sur un modèle expérimental particulier, à savoir le rat placé en isolement temporel (obscurité permanente).

### PROTOCOLE

Des rats mâles Long Evans âgés de un mois sont soumis dès leur arrivée au laboratoire à un cycle lumineux de 12h de lumière par 24h (LD 12 : 12).

Après 2 à 3 semaines d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).

Dès que les rythmes enregistrés témoignent d'un entraînement stable par le cycle lumineux LD 12 : 12, les rats sont mis en obscurité permanente (DD).

Deux à trois semaines plus tard, lorsque le libre-cours (rythme reflétant celui de l'horloge endogène) est clairement établi, les rats reçoivent une administration quotidienne de la molécule à tester.
Les observations sont réalisées grâce à la visualisation des rythmes d'activité :
- entraînement des rythmes d'activité par le rythme lumineux,
- disparition de l'entraînement des rythmes en obscurité permanente,
- entraînement par l'administration quotidienne de la molécule ; effet transitoire ou durable.
Un logiciel permet :
- de mesurer la durée et l'intensité de l'activité, la période du rythme chez les animaux en libre cours et pendant le traitement,
- de mettre éventuellement en évidence par analyse spectrale l'existence de composants circadiens et non circadiens (ultradiens par exemple).

### RESULTATS

II apparaît clairement que les composés de l'invention permettent d'agir de façon puissante sur le rythme circadien via le système mélatoninergique.

### EXEMPLE E : ACTIVITE ANTIARYTHMIQUE

### PROTOCOLE

- (Ref:: LAWSON J.W. et al. J. Pharmacol. Expert. Therap. 160 : 22-31, 1968)

La substance testée est administrée en intrapéritonéal à un groupe de 3 souris 30 min avant l'exposition à une anesthésie par le chloroforme. Les animaux sont ensuite observés pendant 15 min. L'absence d'enregistrement d'arythmies et de fréquences cardiaques supérieures à 200 battements / min (témoin : 400-480 battements / min) chez deux animaux au moins indique une protection significative.

### EXEMPLE F : ACTIVITE ANTI-AGREGANTE PLAQUETTAIRE

### PROTOCOLE

- (Ref. :: Bertele V. et al. Science. 220 : 517-519, 1983
Ibid, Eur. J. Pharmacol. 85 : 331-333, 1982)

Les composés de l'invention (100 µg/ml) sont testés pour leur capacité d'inhiber l'agrégation plaquettaire irréversible induite par l'arachidonate de sodium (50 µg/ml) dans du plasma de lapin enrichi en plaquettes.

Une inhibition de plus de 50 % de l'agrégation maximum indique une activité significative pour les composés de l'invention.

Ce test *in vitro* montre que les composés de l'invention sont de bons candidats pour le traitement des maladies cardiovasculaires, notamment les thromboses

### EXEMPLE G : PROLONGATION DU TEMPS DE SAIGNEMENT

### PROTOCOLE

- (Ref. :: Djana E. et al. Thrombosis Research. 15 : 191-197, 1979)
Butler K.D. et al. Thromb. Haemostasis. 47 : 46-49, 1982)

Les composés à tester sont administrés par voie orale (100 mg/kg) à un groupe de 5 souris 1h avant le sectionnement standardisé du bout de chaque queue (0,5 mm).

Les souris sont immédiatement suspendues verticalement, les queues étant immergées de 2 cm dans un tube à essai contenant une solution saline isotonique à 37°C.

Le temps requis pour que le saignement cesse pendant une période de 15 secondes est alors déterminé.

Une prolongation de plus de 50 % du temps de saignement relative à un groupe d'animaux contrôle est considérée comme significative pour les composés de l'invention.

Ce test *in vivo* confirme l'intérêt des composés de l'invention pour le traitement des pathologies cardiovasculaires puisque les composés de l'invention prolongent le temps de saignement.

### EXEMPLE H : TEST D'HYPOXIE HYPOBARE

### PROTOCOLE

- (Ref. :: Gotti B., et Depoortere H., Circ. Cerebrale, Congrès de Circulation Cérébrale, Toulouse, 105-107, 1979)

Les composés à tester sont administrés par voie intrapéritonéale (100 mg/kg) à un groupe de 3 souris 30 minutes avant d'être placés dans une chambre à la pression hypobare de 20 cm Hg.

La prolongation du temps de survie par rapport à un groupe d'animaux traités avec le véhicule de plus de 100 % en absence d'effet dépresseur du système nerveux central indique une activité cérébroprotective des composés de l'invention.

### EXEMPLE I : COMPOSITION PHARMACEUTIQUE : COMPRIMES

1000 comprimés dosés à 5 mg de N-[2-(7-méthoxy 8-allyl napht-1-yl)éthyl]acétamide

| | |
|---|---|
| N-[2-(7-méthoxy 8-allyl napht-1-yl)éthyl]acétamide | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I) : 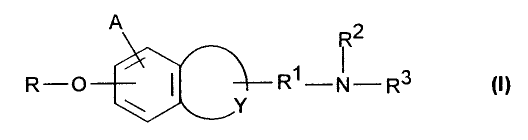 dans laquelle :
- R¹ représente une chaîne (C₁-C₄) alkylène non substituée ou substituée par un radical choisi parmi alkyle, hydroxy, alkoxycarbonyle et carboxyle;
- R² représente un atome d'hydrogène ou un alkyle;
- R³ représente :
. soit un groupement de formule R³¹ 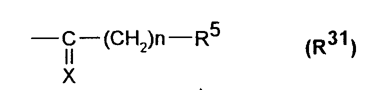 dans lequel n représente zéro ou un nombre entier de 1 à 3, X représente un soufre ou un oxygène et R⁵ représente un atome d'hydrogène, un alkyle non substitué ou substitué, un alcényle, un alcynyle, un cycloalkyle non substitué ou substitué, un dicycloalkylalkyle non substitué ou substitué,
. soit un groupement de formule R³² : 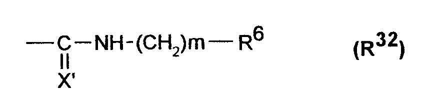 dans lequel X' représente un atome d'oxygène ou de soufre,
m représente zéro ou un nombre entier de 1 à 3
et R⁶ représente un atome d'hydrogène, un alkyle non substitué ou substitué, un alcényle ou un alcynyle, étant entendu que R⁶ peut également représenter un cycloalkyle non substitué ou substitué ou un dicycloalkylalkyl non substitué ou substitué lorsque X' est un oxygène;
- A représente un radical choisi parmi un alkyle non substitué ou substitué et un groupement A' choisi parmi un alcényle ou un alcynyle,
- R représente un groupement choisi parmi un alkyle non substitué ou substitué, un alcényle, un alcynyle, un cycloalkyle non substitué ou substitué, un cycloalkylalkyle non substitué ou substitué, un aryle non substitué ou substitué, un arylalkyle non substitué ou substitué, un diarylalkyle non substitué ou substitué, un cycloalcényle et un cycloalcénylalkyle,
Y forme avec le noyau benzo auquel il est lié un groupement Y¹ qui
- soit représente un groupement choisi parmi naphtalène, benzofurane, benzothiophène et indole, dans le cas où A représente un groupement A',
- soit représente un groupement naphtalène, dans le cas où A représente un radical alkyle non substitué ou substitué,
Y¹ étant éventuellement partiellement hydrogéné,
étant entendu que :
- l'expression "substitué" affectant le terme "alkyle" signifie que ce groupement est substitué par un ou plusieurs radicaux choisis parmi halogène, hydroxy et alkoxy,
- l'expression "substitué" affectant les termes "cycloalkyle", "cycloalkylalkyle" et "dicycloalkylalkyle" signifie que ces groupements sont substitués sur la partie cycloalkyle par un ou plusieurs groupements choisis parmi halogène, alkyle, alkoxy, hydroxy et oxo,
- l'expression "substitué" affectant les termes "aryle", "arylalkyle" et "diarylalkyle" signifie que ces groupements sont substitués sur le noyau aromatique par un ou plusieurs radicaux choisis parmi halogène, alkyle, alkyle substitué par un ou plusieurs halogènes, alkoxy et hydroxy,
- les termes "alkyle" et "alkoxy" désignent des radicaux linéaires ou ramifiés comportant de 1 à 6 atomes de carbone,
- les termes "alcényle" et "alcynyle" désignent des radicaux linéaires ou ramifiés insaturés de 2 à 6 atomes de carbone,
- le terme "cycloalkyle" désigne un groupement cyclique de 3 à 8 atomes de carbone, saturé,
- le terme "cycloalcényle" désigne un groupement cyclique insaturé de 3 à 8 atomes de carbone,
- le terme "aryle" désigne un groupement phényle ou naphtyle,
leurs énantiomères et diastéréoisomères,
et leurs sels d'addition à une base pharmaceutiquement acceptable.

2. Composé selon la revendication 1 qui est le N-[2-(7-méthoxy 8-allyl napht-1-yl)éthyl]acétamide.

3. Composé selon la revendication 1 qui est le N-[2-(7-méthoxy 8-propyl 1,2,3,4-tétrahydronapht-1-yl)éthyl]acétamide et ses énantiomères.

4. Composé selon la revendication 1 qui est le N-[2-(7-méthoxy 8-propyl napht-1-yl)éthyl]acétamide.

5. Composé selon la revendication 1 qui est le N-[2-(7-méthoxy 8-allyl napht-1-yl)éthyl]butyramide.

6. Composé selon la revendication 1 qui est le N-[2-(7-méthoxy 8-allyl napht-1-yl)éthyl]cyclopropanecarboxamide.

7. Composé selon la revendication 1 qui est le N-[2-(7-méthoxy 8-allyl napht-1-yl)éthyl[cyclobutanecarboxamide.

8. Procédé de préparation des composés de formule (I) selon la revendication 1 caractérisé en ce qu'on fait réagir un composé de formule (II) : 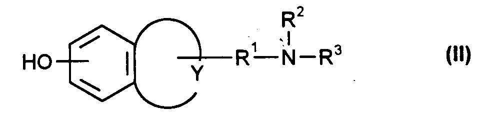 dans laquelle R¹, R² et R³ et Y sont tels que définis dans la revendication 1 avec un composé de formule (III/a) :
A―Z (III/a)
où A a la même définition que dans la revendication 1 et Z représente un groupement partant, par exemple un atome d'halogène ou un groupement tosyle, afin d'obtenir un composé de formule (IV) : 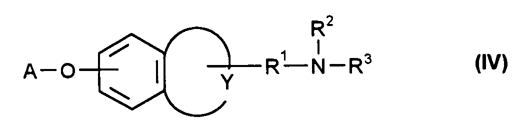 dans laquelle A, R¹, R², R³ et Y sont tels que définis précédemment, ce composé de formule (IV) subissant sous reflux une réaction de transposition qui conduit au composé de formule (V) : 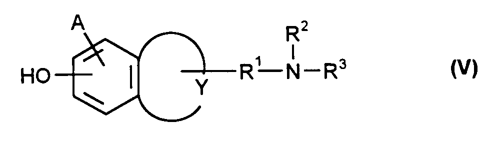 dans laquelle A, R¹, R², R³ et Y sont tels que définis précédemment, ce composé de formule (V) étant ensuite alkylé par un radical de formule R avec R tel que défini dans la revendication 1, pour obtenir un composé de formule (I) : 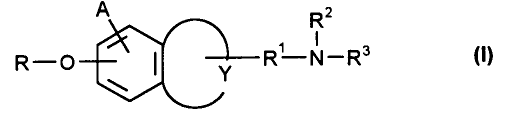 dans laquelle A, R, R¹, R², R³ et Y sont tels que définis précédemment,
composés de formule (I) pouvant être, si on le désire,
- purifiés suivant une ou plusieurs méthodes de purification choisies parmi la cristallisation, la chromatographie sur gel de silice, l'extraction, la filtration, et le passage sur charbon ou résine,
- séparés, le cas échéant, sous forme pure ou sous forme de mélange, en leurs éventuels énantiomères ou diastéréoisomères,
- ou salifiés par une base pharmaceutiquement acceptable.

9. Procédé de préparation des composés de formule (I/a), cas particulier des composés de formule (I) selon la revendication 1 : 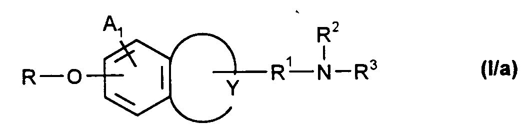 dans laquelle Y, R, R¹, R², R³ sont tels que définis dans la revendication 1 et A₁ est un radical alkyle,
caractérisé en ce qu'un composé de formule (I/b) : 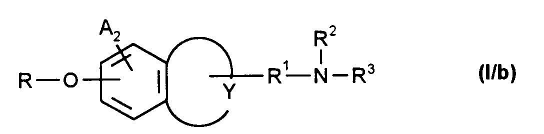 dans laquelle Y, R, R¹, R² et R³ sont tels que précédemment et A₂ est un radical alcényle, est hydrogéné.

10. Procédé de préparation des composés de formule (I) selon la revendication 1 caractérisé en ce qu'on fait réagir un composé de formule (VI) : 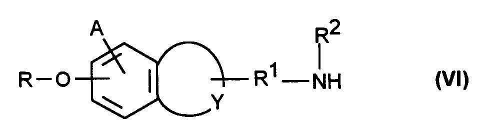 dans laquelle A, R, R¹, R² et Y sont telles que définis dans la revendication 1,
a) avec un chlorure d'acyle de formule (VII) : 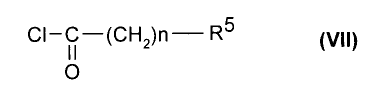 dans laquelle n et R⁵ sont tels que définis dans la revendication 1, ou avec l'anhydride d'acide (mixte ou symétrique) correspondant,
b) ou bien avec un iso(thio)cyanate de formule (VII) :
X=C=N―(CH₂)m―R⁶ (VIII)
avec X, m et R⁶ tels que définis dans la revendication 1, afin d'obtenir, respectivement :
a) le composé de formule (I/c) : 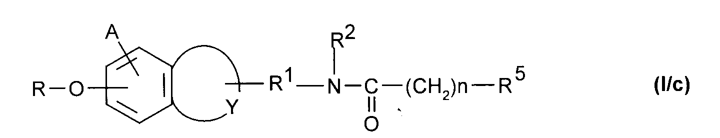 dans laquelle A, R, Y, R¹, R², R⁵ et n sont tels que définis précédemment,
ou b) le composé de formule (I/d) : 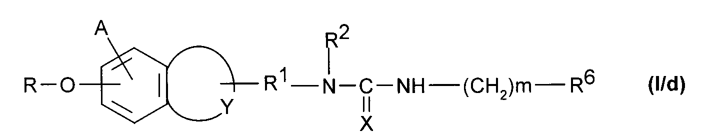 dans laquelle A, R, Y, R¹, R², R⁶, X et m sont tels que définis précédemment,
le composé de formule (I/c) pouvant être soumis à un agent de thionation, pour obtenir les composés de formule (I/e) : 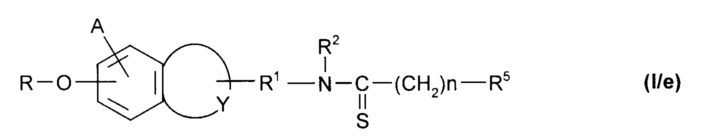 dans laquelle A, R, Y, R¹, R², R⁵ et n sont tels que définis précédemment,
les composés de formule (I/c), (I/d), et (I/e) formant l'ensemble des composés de formule (I),
les composés de formule (I) selon la revendication 1 pouvant être, si on le désire,
- purifiés suivant une ou plusieurs méthodes de purification choisies parmi la cristallisation, la chromatographie sur gel de silice, l'extraction, la filtration, et le passage sur charbon ou résine,
- séparés, le cas échéant, sous forme pure ou sous forme de mélange, en leurs éventuels énantiomères ou diastéréoisomères,
- ou salifiés par une base pharmaceutiquement acceptable.

11. Compositions pharmaceutiques contenant les produits de formule (I) selon la revendication 1 ou le cas échéant un de leurs sels d'addition à une base pharmaceutiquement acceptable en combinaison avec un ou plusieurs excipients pharmaceutiquement acceptables.

12. Compositions selon la revendication 11 utiles dans le traitement des troubles du système mélatoninergique.

## Claims

1. Compounds of formula (I): 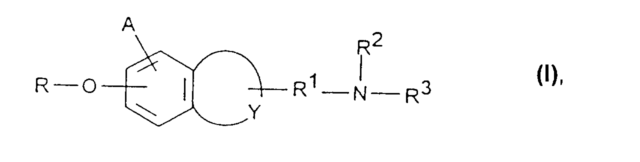 wherein :
- R¹ represents a (C₁-C₄)alkylene chain unsubstituted or substituted by a radical selected from alkyl, hydroxy, alkoxycarbonyl and carboxyl ;
- R² represents a hydrogen atom or an alkyl group ;
- R³ represents :
· either a group of formula R³¹ : 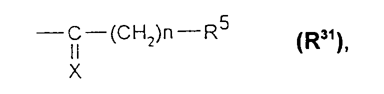 wherein n represents zero or an integer from 1 to 3, X represents a sulphur atom or an oxygen atom and R⁵ represents a hydrogen atom, an unsubstituted or substituted alkyl group, an alkenyl group, an alkynyl group, an unsubstituted or substituted cycloalkyl group or an unsubstituted or substituted dicycloalkylalkyl group,
· or a group of formula R³² : 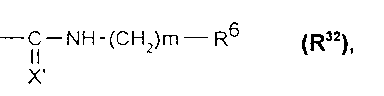 wherein X' represents an oxygen atom or a sulphur atom,
m represents zero or an integer from 1 to 3
and R⁶ represents a hydrogen atom, an unsubstituted or substituted alkyl group, an alkenyl group or an alkynyl group, it being understood that R⁶ may also represent an unsubstituted or substituted cycloalkyl group or an unsubstituted or substituted dicycloalkylalkyl group when X' is an oxygen atom;
- A represents a radical selected from unsubstituted or substituted alkyl and a group A' selected from alkenyl and alkynyl;
- R represents a group selected from unsubstituted or substituted alkyl, alkenyl, alkynyl, unsubstituted or substituted cycloalkyl, unsubstituted or substituted cycloalkylalkyl, unsubstituted or substituted aryl, unsubstituted or substituted arylalkyl, unsubstituted or substituted diarylalkyl, cycloalkenyl and cycloalkenylalkyl;
Y, together with the benzene ring to which it is bonded, forms a group Y¹, which
- either represents a group selected from naphthalene, benzofuran, benzothiophene and indole when A represents a group A',
- or represents a naphthalene group when A represents an unsubstituted or substituted alkyl radical,
Y¹ being optionally partially hydrogenated,
it being understood that:
- the expression "substituted" applied to the term "alkyl" indicates that such a group is substituted by one or more radicals selected from halogen, hydroxy and alkoxy,
- the expression "substituted" applied to the terms "cycloalkyl", "cycloalkylalkyl" and "dicycloalkylalkyl" indicates that such groups are substituted on the cycloalkyl moiety by one or more groups selected from halogen, alkyl, alkoxy, hydroxy and oxo,
- the expression "substituted" applied to the terms "aryl", "arylalkyl" and "diarylalkyl" indicates that such groups are substituted on the aromatic ring by one or more radicals selected from halogen, alkyl, alkyl substituted by one or more halogen atoms, alkoxy and hydroxy,
- the terms "alkyl" and "alkoxy" denote linear or branched radicals having from 1 to 6 carbon atoms,
- the terms "alkenyl" and "alkynyl" denote unsaturated, linear or branched radicals having from 2 to 6 carbon atoms,
- the term "cycloalkyl" denotes a saturated cyclic group having from 3 to 8 carbon atoms,
- the term "cycloalkenyl" denotes an unsaturated cyclic group having from 3 to 8 carbon atoms,
- the term "aryl" denotes a phenyl or naphthyl group,
enantiomers and diastereoisomers thereof,
and addition salts thereof with a pharmaceutically acceptable base.

2. Compound according to claim 1 which is N-[2-(7-methoxy-8-allyl-naphth-1-yl)ethyl]-acetamide.

3. Compound according to claim 1 which is N-[2-(7-methoxy-8-propyl-1,2,3,4-tetrahydronaphth-1-yl)ethyl]acetamide and its enantiomers.

4. Compound according to claim 1 which is N-[2-(7-methoxy-8-propyl-naphth-1-yl)-ethyl]acetamide.

5. Compound according to claim 1 which is N-[2-(7-methoxy-8-allyl-naphth-1-yl)ethyl]-butyramide.

6. Compound according to claim 1 which is N-[2-(7-methoxy-8-allyl-naphth-1-yl)ethyl]-cyclopropanecarboxamide.

7. Compound according to claim 1 which is N-[2-(7-methoxy-8-allyl-naphth-1-yl)ethyl]-cyclobutanecarboxamide.

8. Process for the preparation of compounds of formula (I) according to claim 1, characterised in that a compound of formula (II) : 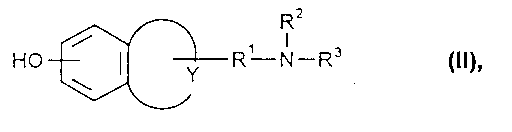 wherein R¹, R², R³ and Y are as defined in claim 1, is reacted with a compound of formula (III/a) :
A―Z (III/a),
wherein A is as defined in claim 1 and Z represents a leaving group, for example a halogen atom or a tosyl group,
to obtain a compound of formula (IV) : 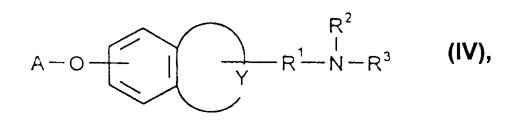 wherein A, R¹, R², R³ and Y are as defined hereinbefore, which compound of formula (IV) undergoes, under reflux, a rearrangement reaction which yields the compound of formula (V) : 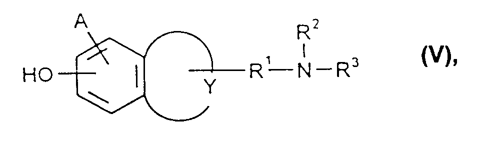 wherein A, R¹, R², R³ and Y are as defined hereinbefore, which compound of formula (V) is then alkylated with a radical of formula R, R being as defined in claim 1, to obtain a compound of formula (I) : 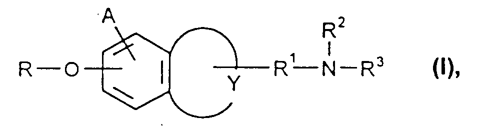 wherein A, R, R¹, R², R³ and Y are as defined hereinbefore,
which compounds of formula (I) may be, if desired,
- purified according to one or more purification methods selected from crystallisation, chromatography on silica gel, extraction, filtration, and passage over carbon or resin,
- separated, where appropriate, in pure form or in the form of a mixture, into their possible enantiomers or diastereoisomers,
- or converted into salts with a pharmaceutically acceptable base.

9. Process for the preparation of compounds of formula (I/a), a particular case of the compounds of formula (I) according to claim 1 : 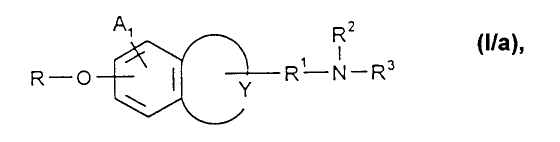 wherein Y, R, R¹, R² and R³ are as defined in claim 1 and A₁ is an alkyl radical, characterised in that a compound of formula (I/b) : 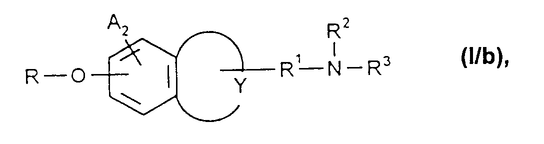 wherein Y, R, R¹, R² and R³ are as hereinbefore and A₂ is an alkenyl radical, is hydrogenated.

10. Process for the preparation of compounds of formula (I) according to claim 1, characterised in that a compound of formula (VI) : 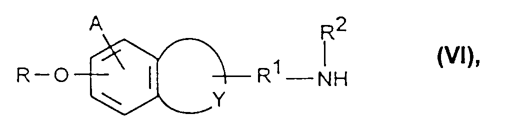 wherein A, R, R¹, R² and Y are as defined in claim 1,
is reacted
a) with an acyl chloride of formula (VII) : 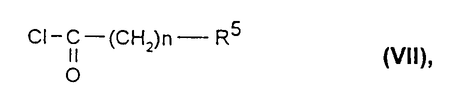 wherein n and R⁵ are as defined in claim 1, or with the corresponding acid anhydride (mixed or symmetrical),
b) or with an iso(thio)cyanate of formula (VIII) :
X=C=N―(CH₂)m―R⁶ (VIII),
wherein X, m and R⁶ are as defined in claim 1,
to obtain, respectively :
a) the compound of formula (I/c) : 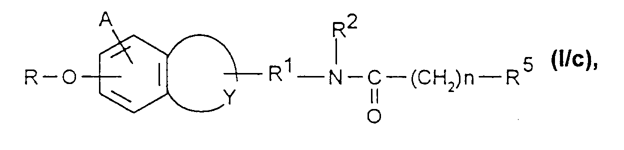 wherein A, R, Y, R¹, R², R⁵ and n are as defined hereinbefore,
or b) the compound of formula (I/d) : 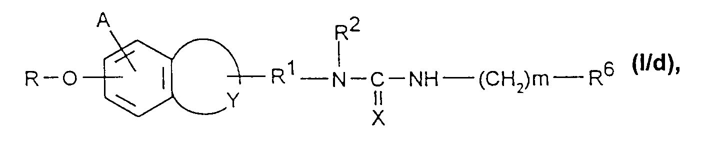 wherein A, R, Y, R¹, R², R⁶, X and m are as defined hereinbefore, which compound of formula (I/c) may be treated with a thionating agent to obtain the compounds of formula (I/e) : 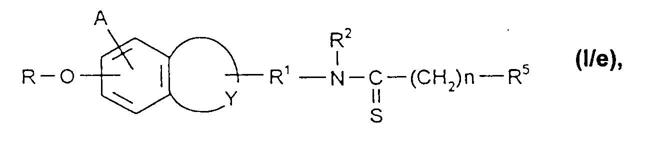 wherein A, R, Y, R¹, R², R⁵ and n are as defined hereinbefore,
the compounds of formulae (I/c), (I/d) and (I/e) constituting the totality of the compounds of formula (I),
which compounds of formula (I) according to claim 1 may be, if desired,
- purified according to one or more purification methods selected from crystallisation, chromatography on silica gel, extraction, filtration, and passage over carbon or resin,
- separated, where appropriate, in pure form or in the form of a mixture, into their possible enantiomers or diastereoisomers,
- or converted into salts with a pharmaceutically acceptable base.

11. Pharmaceutical compositions comprising the compounds of formula (I) according to claim 1 or, where appropriate, an addition salt thereof with a pharmaceutically acceptable base, in combination with one or more pharmaceutically acceptable excipients.

12. Compositions according to claim 11 for use in the treatment of disorders of the melatoninergic system.

## Patentansprüche

1. Verbindungen der Formel (I): 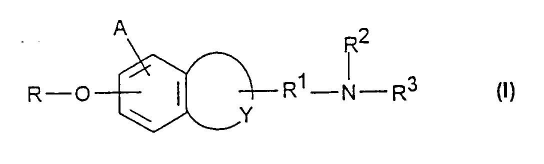 in der:
- R¹ eine (C₁ -C₄)-Alkylenkette, die nichtsubstituiert oder durch eine Gruppe ausgewählt aus Alkyl, Hydroxy, Alkoxycarbonyl und Carboxyl substituiert ist;
- R² ein Wasserstoffatom oder Alkyl;
- R³:
. entweder eine Gruppe der Formel R³¹ 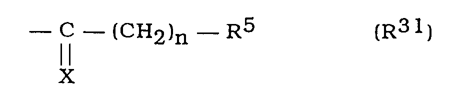 in der n Null oder eine ganze Zahl mit einem Wert von 1 bis 3, X Schwefel oder Sauerstoff und R⁵ ein Wasserstoffatom, nichtsubstituiertes oder substituiertes Alkyl, Alkenyl, Alkinyl, nichtsubstituiertes oder substituiertes Cycloalkyl oder nichtsubstituiertes oder substituiertes Dicycloalhylalkyl darstellen,
. oder eine Gruppe der Formel R³²: 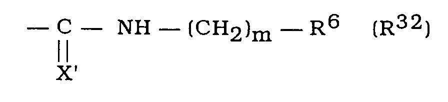 in der X' ein Sauerstoff- oder Schwefelatom,
m Null oder eine ganze Zahl mit einem Wert von 1 bis 3 und
R⁶ ein Wasserstoffatom, nichtsubstituiertes oder substituiertes Alkyl, Alkenyl oder Alkinyl mit der Maßgabe darstellen, daß R⁶ auch nichtsubstituiertes oder substituiertes Cycloalkyl oder nichtsubstituiertes oder substituiertes Dicycloalkyl darstellen kann, wenn X' ein Sauerstoffatom bedeutet;
- A eine Gruppe ausgewählt aus nichtsubstituiertem oder substituiertem Alkyl und einer Gruppe A' ausgewählt aus Alkenyl oder Alkinyl,
- R eine Gruppe ausgewählt aus nichtsubstituiertem oder substituiertem Alkyl, Alkenyl, Alkinyl, nichtsubstituiertem oder substituiertem Cycloalkyl, nichtsubstituiertem oder substituiertem Cycloalkylalkyl, nichtsubstituiertem oder substituiertem Aryl, nichtsubstituiertem oder substituiertem Arylalkyl, nichtsubstituiertem oder substituiertem Diarylalkyl, Cycloalkenyl und Cycloalkenylalkyl bedeuten,
Y mit dem Benzokern, an den es gebunden ist, eine Gruppe Y¹ bildet, welche
- entweder eine Gruppe ausgewählt aus Naphthalin, Benzofuran, Benzothiophen und Indol darstellt, wenn A eine Gruppe A' bedeutet,
- oder eine Naphthalingruppe darstellt, wenn A eine nichtsubstituierte oder substituierte Alkylgruppe bedeutet, wobei
Y¹ gegebenenfalls teilweise hydriert ist,
wobei es sich versteht, daß:
- der Begriff "substituiert" bezüglich des Begriffs "Alkyl" bedeutet, daß diese Gruppe durch eine oder mehrere Gruppen ausgewählt aus Halogen, Hydroxy und Alkoxy substituiert ist,
- der Begriff "substituiert" bezüglich der Begriffe "Cycloalkyl", "Cycloalkylalkyl" und "Dicycloalkylalkyl" bedeutet, daß diese Gruppen am Cycloalkylteil durch eine oder mehrere Gruppen ausgewählt aus Halogen, Alkyl, Alkoxy, Hydroxy und Oxo substituiert sind,
- der Begriff "substituiert" bezüglich der Begriffe "Aryl", "Arylalkyl" und "Diarylalkyl" bedeutet, daß diese Gruppen am aromatischen Kern durch eine oder mehrere Gruppen ausgewählt aus Halogen, Alkyl, durch ein oder mehrere Halogene substituiertes Alkyl, Alkoxy und Hydroxy substituiert sind,
- die Begriffe "Alkyl" und "Alkoxy" für geradkettige oder verzweigte Gruppen mit 1 bis 6 Kohlenstoffatomen stehen,
- die Begriffe "Alkenyl" und "Alkinyl" für ungesättigte geradkettige oder verzweigte Gruppen mit 2 bis 6 Kohlenstoffatomen stehen,
- der Begriff "Cycloalkyl" für eine gesättigte cyclische Gruppe mit 3 bis 8 Kohlenstoffatomen steht,
- der Begriff "Cycloalkenyl" für eine ungesättigte cyclische Gruppe mit 3 bis 8 Kohlenstoffatomen steht und
- der Begriff "Aryl" für eine Phenyl- oder Naphthylgruppe steht,
deren Enantiomere und Diastereoisomere
und deren Additionssalze mit einer pharmazeutisch annehmbaren Base.

2. Verbindung nach Anspruch 1, nämlich N-[2-(7-Methoxy-8-allyl-naphth-1-yl)-ethyl]-acetamid.

3. Verbindung nach Anspruch 1, nämlich N-[2-(7-Methoxy-8-propyl-1,2,3,4-tetrahydronaphth-1-yl)-ethyl]-acetamid und dessen Enantiomere.

4. Verbindung nach Anspruch 1, nämlich N-[2-(7-Methoxy-8-propyl-naphth-1-yl)-ethyl]-acetamid.

5. Verbindung nach Anspruch 1, nämlich N-[2-(7-Methoxy-8-allyl-naphth-1-yl)-ethyl]-butyramid.

6. Verbindung nach Anspruch 1, nämlich N-[2-(7-Methoxy-8-allyl-naphth-1-yl)-ethyl]-cyclopropancarboxamid.

7. Verbindung nach Anspruch 1, nämlich N-[2-(7-Methoxy-8-allyl-naphth-1-yl)-ethyl]-cyclobutancarboxamid.

8. Verfahren zur Herstellung der Verbindungen der Formel (I) nach Anspruch 1, dadurch gekennzeichnet, daß man eine Verbindung der Formel (II): 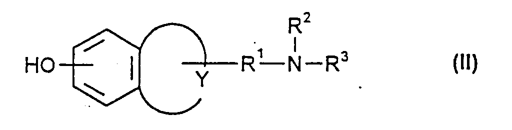 in der R¹, R², R³ und Y die in Anspruch 1 angegebenen Bedeutungen besitzen, mit einer Verbindung der Formel (III/a):
A - Z (III/a)
in der A die in Anspruch 1 angegebenen Bedeutungen besitzt und Z eine austretende Gruppe, beispielsweise ein Halogenatom oder eine Tosylgruppe darstellt, umsetzt zur Bildung einer Verbindung der Formel (IV): 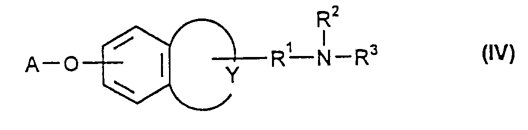 in der A, R¹, R², R³ und Y die oben angegebenen Bedeutungen besitzen, welche Verbindung der Formel (IV) am Rückfluß einer Umlagerungsreaktion unterliegt, welche zu der Verbindung der Formel (V) führt: 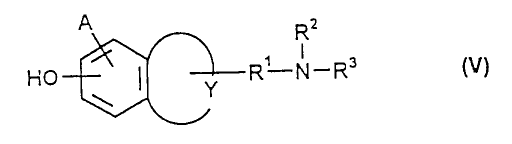 In der A, R¹, R², R³ und Y die oben angegebenen Bedeutungen besitzen, welche Verbindung der Formel (V) anschließend mit einer Gruppe der Formel R, worin R die in Anspruch 1 angegebenen Bedeutungen besitzt, alkyliert wird zur Bildung einer Verbindung der Formel (I); 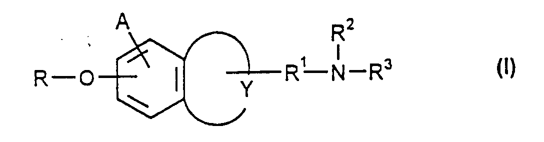 in der A, R, R¹, R², R³ und Y die oben angegebenen Bedeutungen besitzen, welche Verbindungen der Formel (I) gewünschtenfalls:
- mit Hilfe einer oder mehrerer Reinigungsmethoden ausgewählt aus Kristallisation, Chromatographie über Kieselgel, Extraktion, Filtration und Überführung über Aktivkohle oder ein Harz gereinigt werden können,
- gegebenenfalls in ihre eventuellen Enantiomeren oder Diastereoisomeren in reiner Form oder in Form einer Mischung getrennt werden können
- oder mit einer pharmazeutisch annehmbaren Base in die Salze überführt werden können.

9. Verfahren zur Herstellung der Verbindungen der Formel (I/a), einem Sonderfall der Verbindungen der Formel (I) nach Anspruch 1: 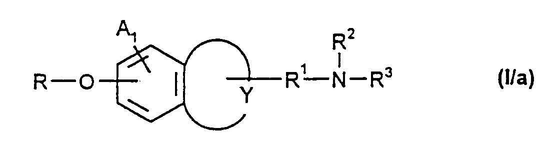 in der Y, R, R¹, R² und R³ die in Anspruch 1 angegebenen Bedeutungen besitzen und A₁ eine Alkylgruppe darstellt,
dadurch gekennzeichnet, daß man eine Verbindung der Formel (I/b): 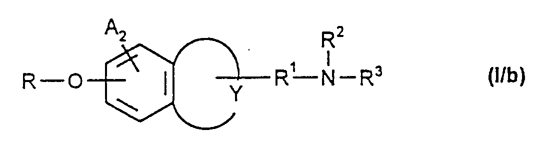 in der Y, R, R¹, R² und R³ die oben angegebenen Bedeutungen besitzen und A₂ eine Alkenylgruppe darstellt, hydriert.

10. Verfahren zur Herstellung der Verbindungen der Formel (I) nach Anspruch 1, dadurch gekennzeichnet, daß man eine Verbindung der Formel (VI): 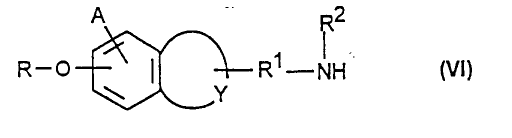 in der A, R, R¹, R² und Y die in Anspruch 1 angegebenen Bedeutungen besitzen,
a) mit einem Acylchlorid der Formel (VII): 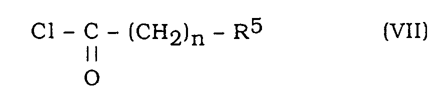 in der n und R⁵ die in Anspruch 1 angegebenen Bedeutungen besitzen, oder mit einem entsprechenden (gemischten oder symmetrischen) Säureanhydrid,
b) oder mit einem Iso(thio)cyanat der Formel (VIII):
X = C = N - (CH₂)m - R⁶ (VIII)
worin X, m und R⁶ die in Anspruch 1 angegebenen Bedeutungen besitzen, umsetzt, so daß man:
a) die Verbindung der Formel (I/c): 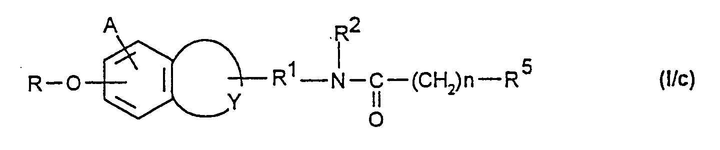 in der A, R, Y, R¹, R², R⁵ und n die oben angegebenen Bedeutungen besitzen, beziehungsweise
b) die Verbindung der Formel (I/d): 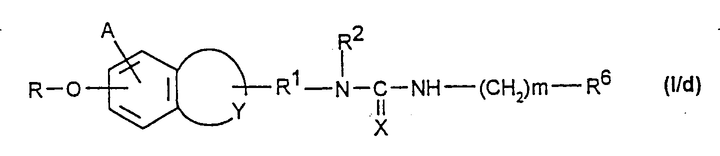 in der A, R, Y, R¹, R², R⁶, X und m die oben angegebenen Bedeutungen besitzen, erhält,
worauf man die Verbindung der Formel (I/c) mit einem Thionierungsmittel behandeln kann zur Bildung der Verbindungen der Formel (I/e): 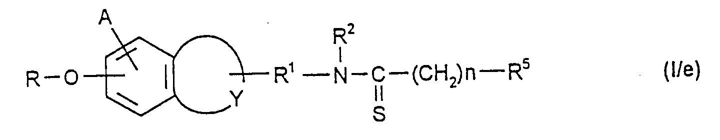 in der A, R, Y, R¹, R², R⁵ und n die oben angegebenen Bedeutungen besitzen, wobei die Verbindungen der Formeln (I/c), (I/d) und (I/e) die Gesamtheit der Verbindungen der Formel (I) bilden,
welche Verbindungen der Formel (I) nach Anspruch 1 gewünschtenfalls:
- mit Hilfe einer oder mehrerer Reinigungsmethoden ausgewählt aus Kristallisation, Chromatographie über Kieselgel, Extraktion, Filtration und Überführung über Aktivkohle oder ein Harz gereinigt werden können,
- gegebenenfalls in ihre eventuellen Enantiomeren oder Diastereoisomeren in reiner Form oder in Form einer Mischung getrennt werden können,
- oder mit einer pharmazeutisch annehmbaren Base in ihre Salze überführt werden können.

11. Pharmazeutische Zubereitungen enthaltend die Produkte der Formel (I) nach Anspruch 1 oder gegebenenfalls eines ihrer Additionssalze mit einer pharmazeutisch annehmbaren Base in Kombination mit einem oder mehreren pharmazeutisch annehmbaren Trägermaterialien.

12. Zubereitungen nach Anspruch 11 zur Behandlung von Störungen des melatoninergischen Systems.
